(19) Europäisches Patentamt
European Patent Office
Office européen des brevets

(11) Numéro de publication: **0 402 246 B1**

(12) **FASCICULE DE BREVET EUROPEEN**

(45) Date de publication de fascicule du brevet: **24.08.94**

(21) Numéro de dépôt: **90401532.8**

(22) Date de dépôt: **06.06.90**

(51) Int. Cl.5: **C07D 277/30**, A01N 43/78, C07D 277/36, C07D 277/32, C07D 263/32, A01N 43/76, C07D 417/04, C07D 277/24

(54) **Nouveaux thiazolylalkoxyacrylates, leur procédé de préparation, leur application comme fongicides et leurs intermédiaires de préparation.**

(30) Priorité: **06.06.89 FR 8907438**

(43) Date de publication de la demande:
**12.12.90 Bulletin 90/50**

(45) Mention de la délivrance du brevet:
**24.08.94 Bulletin 94/34**

(84) Etats contractants désignés:
**AT BE CH DE DK ES FR GB GR IT LI LU NL SE**

(56) Documents cités:
**EP-A- 0 178 826**
**EP-A- 0 256 667**
**EP-A- 0 299 694**

(73) Titulaire: **ROUSSEL-UCLAF**
**35, Boulevard des Invalides**
**F-75007 Paris (FR)**

(72) Inventeur: **Brayer, Jean-Louis**
**42, rue Jules Dubrulle**
**F-60440 Nanteuil le Haudoin (FR)**
Inventeur: **Cadiergue, Joseph**
**6, rue Jules Princet**
**F-93600 Aulnay sous Bois (FR)**
Inventeur: **Mourioux, Gilles**
**12, Allée des Micocouliers**
**F-13420 Gemenos (FR)**
Inventeur: **Tessier, Jean**
**30, rue Jean Moulin**
**F-94300 Vincennes (FR)**

(74) Mandataire: **Tonnellier, Marie-José et al**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 402 246 B1

## Description

La présente invention concerne de nouveaux thiazolylalkoxyacrylates, leur procédé de préparation, leur application come fongicides et leurs intermédiaires de préparation.

Le document EP-A-0 256 667 décrit des dérivés de phénylméthoxyacrylates et leur application comme fongicides.

L'invention a pour objet les composés de formule générale (I) :

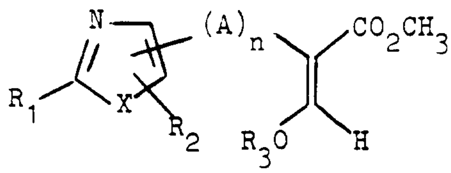

(I)

dans lesquels

- $R_1$ et $R_2$ identiques ou différents représentent un atome d'hydrogène, un atome d'halogène, un radical $CF_3$, un radical alkyle, alkényle, alkynyle ou thioalkyle renfermant jusqu'à 8 atomes de carbone, un radical alkoxy, thioalkoxy ou $SO_2$alkyle renfermant jusqu'à 8 atomes de carbone, un radical aryle, aryloxy ou thioaryle renfermant jusqu'à 18 atomes de carbone éventuellement substitué, un radical hétéroaryle ou hétéroaryloxy éventuellement substitué ou un radical hétérocyclique à 5 ou 6 chaînons éventuellement substitué,
- $R_3$ représente un radical alkyle, alkényle ou alkynyle renfermant jusqu'à 8 atomes de carbone ou un radical aryle renfermant jusqu'à 18 atomes de carbone ou un radical alkoxyalkoxyalkyle renfermant jusqu'à 14 atomes de carbone,
- X représente un atome d'oxygène ou de soufre ou un radical $NR_4$ dans lequel $R_4$ représente un atome d'hydrogène ou un radical alkyle, alkényle ou alkynyle, $SO_2$-alkyle, $SO_2$-alkényle ou $SO_2$-alkynyle renfermant jusqu'à 8 atomes de carbone ou un radical aryle ou $SO_2$-aryle renfermant jusqu'à 18 atomes de carbone éventuellement substitué,
- n représente le nombre O ou le nombre 1,
- A représente un radical choisi dans le groupe des radicaux suivants :

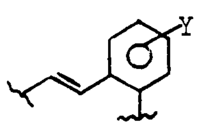 ; 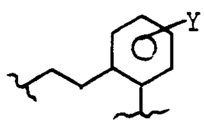 ; 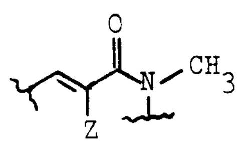 ; 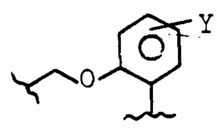

  Y représentant un atome d'hydrogène, un atome d'halogène, un radical $CF_3$, un radical alkyle ou alkoxy renfermant juqu'à 8 atomes de carbone et Z représentant un atome d'hydrogène ou un atome d'halogène,
- la géométrie des doubles liaisons étant E ou Z ou un mélange de géométrie E et Z (au sens de Cahn, Ingold, Prelog).

Dans la définition des différents substituants,

- alkyle représente de préférence un radical méthyle, éthyle, n-propyle, isopropyle, n-butyle, isobutyle, sec-butyle ou tert-butyle,
- alkényle représente de préférence un radical vinyle, allyle ou 1,1-diméthylallyle,
- alkynyle représente de préférence un radical éthynyle ou propynyle,
- aryle représente de préférence un radical phényle,
- hétéroaryle représente de préférence un radical furyle, pyridinyle, pipérazinyle, benzofuranyle, isobenzofuranyle, oxazolyle ou isoxazolyle,
- hétérocyclique représente de préférence un radical morpholinyle, pyranyle, pyridazinyle,
- alkoxyalkoxyalkyle représente de préférence un radical méthoxyéthoxyalkyle ou tout autre radical de formule :

  $(CH_2)_p$-O-$(CH_2)_{p'}$-O-alkyle

  p et p' identiques ou différents représentant les nombres 1, 2, 3 ou 4 et "alkyle" représentant un

radical alkyle renfermant jusqu'à 8 atomes de carbone,

- halogène représente de préférence un atome de fluor ou de chlore.

Lorsque les radicaux aryle, aryloxy, thioaryle, hétérocyclique, hétéroaryle, ou hétéroaryloxy sont substitués, ils portent de préférence des substituants choisis dans le groupe constitué par les radicaux hydroxyles libres, estérifiés ou éthérifiés dans lesquels la partie ester ou éther renferme de 1 à 18 atomes de carbone, comme par exemple le radical acétoxy ou le radical méthoxy, les fonctions cétones et oximes, les radicaux alcoyles linéaires, ramifiés ou cyclisés, saturés ou insaturés, comportant jusqu'à 18 atomes de carbone, par exemple le radical méthyle, éthyle, propyle ou isopropyle, le radical éthényle $-CH=CH_2$ ou le radical éthynyle $-C \equiv CH$, les atomes d'halogène, comme le fluor, le chlore, le brome, les groupements $CF_3$, $SCF_3$, $OCF_3$, $NO_2$, $NH_2$ ou $C \equiv N$.

L'invention a plus particulièrement pour objet :

- les composés de formule (I) dans lesquels le groupement

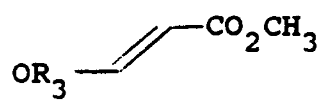

est tel que $CO_2CH_3$ et $OR_3$ sont en conformation trans l'un par rapport à l'autre,
- les composés de formule (I) dans lesquels X représente un atome de soufre ;
- les composés de formule (I) dans lesquels A représente un radical

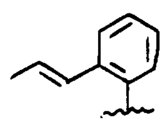

et tout spécialement parmi ceux-ci, les composés dans lesquels la géométrie de la double liaison est E ;
- les composés de formule (I) dans lesquels A représente un radical

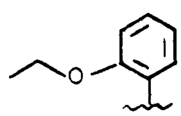

- les composés de formule (I) dans lesquels A représente un radical

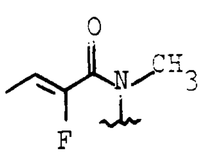

(A1)

ou

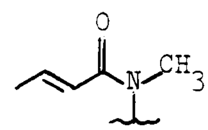

(A2)

et tout spécialement parmi ceux-ci les composés dans lesquels la géométrie de la double liaison est Z pour le radical $A_1$ et E pour le radical $A_2$ ;
- les composés de formule (I) dans lesquels n représente le nombre O ;
- les composés de formule (I) dans lesquels $R_1$ représente un atome d'hydrogène, un atome de chlore, un radical alkyle ou alkényle renfermant jusqu'a 5 atomes de carbone, un radical alkoxy renfermant jusqu'à 5 atomes de carbone ou un radical

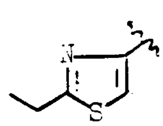

; ;

- les composés de formule (I) dans lesquels $R_2$ représente un atome d'hydrogène ;
- les composés de formule (I) dans lesquels $R_1$ et/ou $R_2$ sont des radicaux aryles éventuellement substitués, ou encore ceux dans lesquels $R_1$ est un radical $SCH_3$ et $R_2$ un radical $CH_3$ et tout spécialement ceux dans lesquels le groupement alkoxyacrylate est en position 5.

Parmi les composés préférés de l'invention, on peut citer les composés dont la préparation est donnée ci-après dans la partie expérimentale et tout spécialement les produits des exemples 1, 5, 11, 13, 14, 15, 16, 17, 20, 28, 29, 30, 31, 32, 33 et 38.

L'invention a également pour objet un procédé de préparation, caractérisé en ce que l'on soumet un composé de formule (II) :

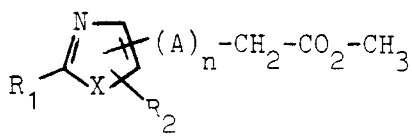

(II)

dans laquelle X, $R_1$, $R_2$, A et n conservent leur signification précédente, à l'action d'une base forte et d'un formiate d'alcoyle ou du N-formyl- imidazole pour obtenir le composé de formule (III) :

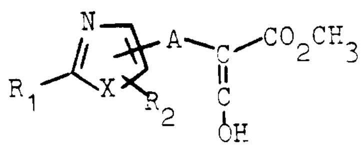

(III)

que l'on soumet à l'action d'un agent capable d'introduire le radical $R_3$ pour obtenir le composé de formule (I) correspondant et sépare le cas échéant les différents isomères.

Dans un mode de réalisation préférée du procédé de l'invention,
- la base utilisée est l'hydrure de sodium,
- le formiate d'alcoyle utilisé est le formiate de méthyle ou d'éthyle,
- l'agent capable d'introduire le radical $R_3$ est un dérivé Hal-$R_3$ dans lequel Hal représente par exemple un atome d'iode ou de chlore ou encore un sulfate $SO_4$-$R_3$,
- le composé de formule (III) n'est pas isolé,
- les différents isomères obtenus peuvent être séparés par chromatographie.

Les composés de formule (II) peuvent être préparés selon les procédés décrits ci-après dans la partie expérimentale qui sont des procédés classiques de la chimie des hétérocycles : ces composés de formule (II), ainsi que les composés de formule (III) obtenus lors de la mise en oeuvre du procédé de préparation des composés de formule (I) sont en eux-mêmes un objet de la présente invention.

L'invention a également pour objet un procédé caractérisé en ce que l'on soumet un composé de formule (IV) :

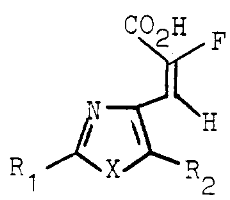

(IV)

dans laquelle X, $R_1$ et $R_2$ conservent leur signification précédente ou un dérivé fonctionnel de ce composé à l'action d'un composé de formule (V) :

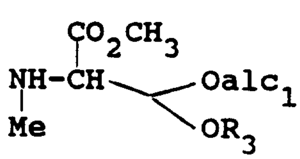

(V)

dans laquelle $R_3$ conserve sa signification précédente et $alc_1$ représente un radical alcoyle renfermant jusqu'à 8 atomes de carbone, pour obtenir le composé de formule (VI) :

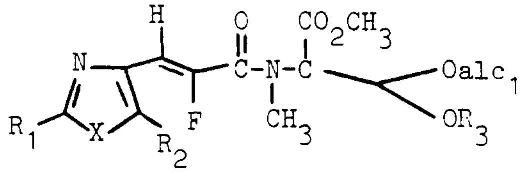

(VI)

que l'on soumet à l'action d'un agent d'élimination du reste de l'alcool $alc_1$-OH, pour obtenir le composé de formule ($I_A$) correspondant :

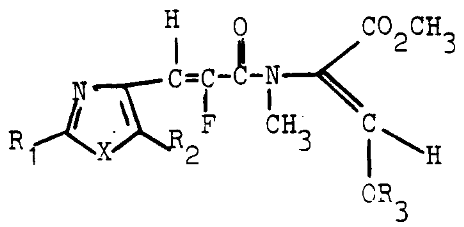

($I_A$)

Dans un mode de réalisation préféré du procédé de l'invention, le dérivé fonctionnel de l'acide (IV) utilisé est un chlorure d'acide. L'élimination du reste de l'alcool $alc_1$-OH a lieu par chauffage en milieu acide par exemple en présence d'acide paratoluènesulfonique.

Les composés de formule (IV) utilisés comme produits de départ peuvent être préparés comme indiqué ci-après dans la partie expérimentale ; ils sont nouveaux et sont en eux-mêmes un objet de la présente invention.

Les composés de formule (V) également utilisés comme produits de départ peuvent être préparés selon le brevet EP 0 178 808.

Les composés de formule (VI) obtenus lors de la mise oeuvre du procédé de l'invention sont nouveaux et sont un objet de la présente invention.

L'invention a également pour objet un procédé, caractérisé en ce que l'on soumet selon la réaction de Wittig-Horner un composé de formule (VII) :

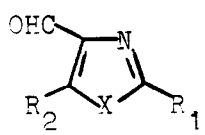

(VII)

dans laquelle X, $R_1$ et $R_2$ conservent leurs significations précédentes à l'action d'un composé de formule (VIII) :

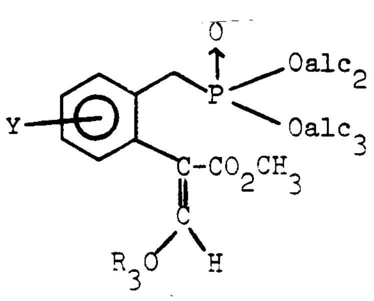

(VIII)

dans laquelle Y et $R_3$ conservent leurs significations précédentes, $alc_2$ et $alc_3$ identiques ou différents représentent un radical alkyle renfermant jusqu'à 8 atomes de carbone, pour obtenir le composé de formule ($I_B$) correspondant :

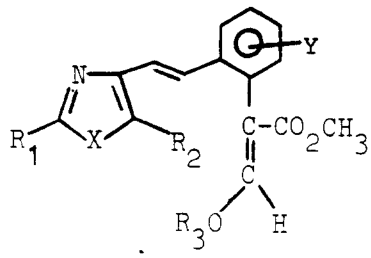

$(I_B)$

que si désiré l'on isole ou si désiré soumet à une hydrogénation catalytique pour obtenir les composés de formule ($I_C$) :

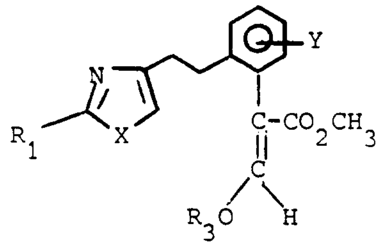

$(I_C)$

Les composés de formule (VIII) peuvent être préparés selon le procédé décrit dans le brevet EP 0 203 606.

L'hydrogénation catalytique conduisant aux produits de formule ($I_C$) peut être effectuée comme indiqué dans le brevet DE 3545318A1.

Dans un mode de réalisation préférée du procédé de l'invention, la réaction du composé (VII) et du composé (VIII) a lieu en présence d'une base forte comme par exemple l'hydrure de sodium, un alcoolate alcalin ou alcalino-terreux, un amidure alcalin ou un dérivé organolithien.

Les composés de formule (I) présentent d'intéressantes propriétés fongicides susceptibles d'être utilisées pour la protection vis-à-vis des champignons pathogènes. Il peut s'agir de la protection des plantes, de la protection des locaux ou des animaux.

Ces propriétés peuvent également être utilisées en hygiène et médecine humaine et animale.

Les composés de l'invention permettent de lutter contre de très nombreux champignons phytopathogènes, notamment contre : Erisyphe graminis, Sphaerotheca macularis, Sphaerotheca fuliginea, Podosphaera leucotricha, Uncinula necator, Helminthosporium spp., Rhynchosporium spp., Pseudocercosporella herpotrichoides and Gaeumannomyces graminis, Ustilago spp., Cercospora arachidicola and Cercosporidium personatum, Cercospora species, Botrytis cinerea, Alternaria spp., Venturia inaequalis, Plasmopara viticola, Bremia lactucae, Peronospora spp., Pseudoperonospora humuli, Pseudoperonospora cubensis, Phytophthora spp. infestans, Phytophthora spp., Thanatephorus cucumeris, Rhizeoctonia spp. ou encore des champignons ou levures intéressant la santé humaine comme Candida albicans ou Trychophyton spp.

L'invention a donc pour objet les compositions fongicides renfermant comme principe actif au moins un composé de formule (I) tel que défini précédemment. L'invention a plus particulièrement pour objet les compositions renfermant les composés dont la préparation chimique est donnée aux exemples 1, 5, 11, 13, 14, 15, 16, 17, 20, 28, 29, 30, 31, 32, 33 et 38.

Les compositions selon l'invention sont préparées selon les procédés usuels de l'industrie agrochimique par exemple. Ces compositions peuvent se présenter sous forme de poudres, granulés, suspensions, émulsions, solutions ou autres préparations employées classiquement pour l'utilisation de ce genre de composés.

Outre le principe actif, ces compositions contiennent en général, un véhicule et/ou un agent tensio-actif ionique ou non ionique, asurant une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselghur ou un solide combustible.

Les compositions peuvent naturellement renfermer un ou plusieurs autres agents pesticides, par exemple un ou plusieurs agents insecticides ou acaricides.

Les exemples suivants illustrent l'invention.

**Préparation 1 : (E)-[méthyl [1-oxo 3-[2-(trifluorométhyl) 4-thiazolyl] 2- propényl] amino] acétate de méthyle**.

**Stade A :** 2,2,2-trifluoroéthanethioamide.

A une solution de 60 g de trifluoroacétamide dans 600 cm3 de tétrahydrofuranne, on ajoute 30 g de pentasulfure de phosphore. Après 1 heure d'agitation à 20/25 °C, on rajoute 35 g de pentasulfure de phosphore et agite 16 heures à 20 °C. On filtre, lave à l'hexane et amène à sec le filtrat. On chromatographie le résidu sur silice en éluant avec hexane-acétate d'éthyle (7-3), on obtient 33 g de produit attendu utilisé tel quel pour le stade suivant.

**Stade B :** 2-(trifluorométhyl) 4-thiazole carboxylate d'éthyle.

A une solution de 26 g du produit obtenu au stade A dans 500 cm3 d'éthanol, on ajoute 39 g de bromopyruvate d'éthyle. On agite 16 heures au reflux. On verse dans 260 cm3 d'eau et ajuste à pH 8 par addition de bicarbonate de sodium. On distille l'éthanol et extrait la fraction aqueuse avec de l'acétate d'éthyle. On évapore à sec et obtient 24 g de produit que l'on chromatographie sur silice en éluant avec hexane-acétate d'éthyle (7-3). On obtient 21 g de produit recherché utilisé tel quel pour le stade suivant.

**Stade C :** 2-(trifluorométhyl) 4-thiazole méthanol.

A un mélange de 25 g de produit obtenu au stade B, 550 cm3 de tétrahydrofuranne et 10,8 g d'hydroborure de sodium, on ajoute au reflux 110 cm3 de méthanol. On agite 45 minutes au reflux puis ramène à 20/25 °C et on ajoute 100 cm3 d'acide chlorhydrique 2N (pH 7). On filtre l'insoluble et évapore le filtrat sous pression réduite. On reprend le résidu avec de l'éther, le sèche et concentre à sec sous pression réduite. On chromatographie le résidu (19 g) sur silice (éluant : hexane-acétate d'éthyle 7-3). On obtient 13,6 g de produit recherché.

**Spectre IR :**
Absence de C=O
OH : 3608 $cm^{-1}$
Système conjugué : 1524 $cm^{-1}$
$CF_3$ probable.

**Stade D :** 2-(trifluorométhyl) 4-thiazole carboxaldéhyde.

On agite 2 heures au reflux un mélange de 13 g du produit obtenu au stade C, 300 cm3 de chlorure de méthylène et 44 g de bioxyde de manganèse. Après ce temps, on rajoute 30 g de bioxyde manganèse et maintient encore 1 heure au reflux. On filtre et évapore à sec le filtrat sous pression réduite (sans chauffer). On chromatographie le résidu (12 g) sur silice en éluant avec hexane-acétate d'éthyle (7-3). On obtient 7,3 g de produit attendu.

**Spectre IR :**

Pas d'OH

| | |
|---|---|
| C=O | : 1710 $cm^{-1}$ |
| CH aldéhyde | 2750 $cm^{-1}$<br>2840 $cm^{-1}$ |
| Système conjugué | 1499 $cm^{-1}$<br>1478 $cm^{-1}$ |

$CF_3$.

**Stade E :** (E) acide [2-(trifluorométhyl) 4-thiazolyl] 2-propénoïque.

A un mélange de 3,62 g du produit obtenu au stade D, 20 cm3 de pyridine et 4,08 g d'acide malonique, on ajoute 1,25 cm3 de pipéridine. On agite 7 heures au reflux. On verse sur un mélange de 35 cm3 d'acide chlorhydrique concentré, 60 cm3 d'eau et glace, On essore et obtient après sèchage 2,9 g de produit recherché. F = 180 ° C.

**Spectre IR :**

Absorption région NH-OH

| | |
|---|---|
| C=O complexe | : 1680 $cm^{-1}$ |
| C=C | : 1635 $cm^{-1}$ |
| Système conjugué | : 1504 $cm^{-1}$ |
| C=CH trans | : 987 $cm^{-1}$ |

**Stade F :** (E)-[méthyl [1-oxo 3-[2-(trifluorométhyl) 4-thiazolyl] 2- propényl] amino] acétate de méthyle.

A une solution de 3,62 g de chlorhydrate de méthylaminoacétate de méthyle, 60 cm3 de chlorure de méthylène et 30 cm3 de diméthylformamide, on ajoute 3,62 $cm_3$ de triéthylamine, agite 15 minutes à 20/25 ° C et filtre l'insoluble. Au filtrat, on ajoute 2,9 g de l'acide obtenu au stade E, 0,1 g de diméthylaminopyridine et une solution de 2,68 g de dicyclohexylcarbodiimide dans 10 cm3 de chlorure de méthylène. On agite pendant 6 heures à 20/25 ° C. On filtre l'insoluble et évapore le filtrat à sec sous pression réduite. On reprend le résidu à l'eau, on essore le produit cristallisé que l'on dissout dans du chlorure de méthylène, filtre l'insoluble, sèche et évapore à sec le filtrat. On chromatographie le résidu sur silice (éluant : hexane-acétate d'éthyle 1-1). On obtient 2,6 g du produit recherché. F = 120 ° C.

**Spectre IR :**

| | |
|---|---|
| $-C(=O)-OCH_3$ | 1749 $cm^{-1}$<br>1438 $cm^{-1}$ |
| $-C=C-C(=O)-N$ | 1652 $cm^{-1}$<br>1616 $cm^{-1}$ |
| HC=CH trans | : 972 $cm^{-1}$ |

**Exemple 1 : (E,Z) 3-méthoxy 2-[méthyl [1-oxo 3-[2-(trifluorométyl) 4- thiazolyl] 2-propényl] amino] 2-propènoate de méthyle.**

**A - Formylation :**

A une suspension de 0,8 g d'hydrure de sodium dans 30 cm3 de diméthylformamide, on ajoute lentement 2,5 g du produit obtenu au stade F de la préparation 1 en solution dans 25 cm3 de diméthylformamide et 9,6 cm3 de formiate de méthyle. Après 1 heure d'agitation à 20/25 °C, on rajoute 4 cm3 de formiate de méthyle et agite 16 heures à 20/25 °C. On coule sur une solution saturée de carbonate de sodium, filtre l'insoluble et extrait le filtrat à l'éther. On acidifie la phase aqueuse, l'extrait avec de l'éther, sèche et évapore à sec sous pression réduite. On obtient 3 g de produit formylé que l'on utilise tel quel pour la suite.

**B - Méthylation :**

A un mélange de 30 cm3 de diméthylformamide et 0,4 g d'hydrure de sodium, on ajoute une solution de 3 g de produit formylé obtenu au stade précédent dans 30 cm3 de diméthylformamide. Après 30 minutes, on ajoute 1 cm3 d'iodure de méthyle. On agite 3 heures à 20/25 °C puis on verse dans de l'eau chlorhydrique, extrait avec de l'éther isopropylique, sèche et évapore à sec sous pression réduite. On chromatographie le résidu (2,9 g) sur silice (éluant : hexane-acétate d'éthyl 3-7). On obtient 0,75 g de produit recherché. F = 104 °C.

| Analyse : $C_{13}H_{13}N_2F_3O_4S$ | | | | | | | | | | |
|---|---|---|---|---|---|---|---|---|---|---|
| Calculé<br>Trouvé | C% | 44,57<br>44,5 | H% | 3,74<br>3,7 | N% | 8,00<br>7,9 | F% | 16,27<br>16,2 | S% | 9,15<br>9,2 |

**Exemple 2 : (E,Z) 3-[[(2-méthoxy) éthoxy] méthoxy] 2-[méthyl-[1-oxo 3-[2- (trifluorométhyl) 4-thiazolyl] 2-propényl] amino]] 2-propénoate de méthyle.**

On opère comme en B de l'exemple 1 à partir de 4,4 g du produit obtenu en A de l'exemple 1 en utilisant 2,1 cm3 de chlorure de méthoxyéthoxyméthyle. On obtient après chromatographie sur silice (éluant : hexane-acétate d'éthyle 7-3), 4,7 g de produit que l'on chromatographie à nouveau sur silice sous pression (éluant : chlorure de méthylène-tétrahydrofuranne 95-5). On recueille 3,3 g du produit recherché. F = 76 °C.

| Analyse : $C_{16}H_{19}F_3N_2O_6S$ | | | | | | | | | | |
|---|---|---|---|---|---|---|---|---|---|---|
| Calculé<br>Trouvé | C% | 45,28<br>45,0 | H% | 4,51<br>4,5 | N% | 6,6<br>6,5 | F% | 13,43<br>13,8 | S% | 7,56<br>7,6 |

**Préparation A : 4-bromo 3-oxo butyrate de méthyle.**

On refroidit à 0 °C un mélange de 115 g d'acétylacétate de méthyle et 200 cm3 d'éther éthylique et ajoute en 1 heure 54 cm3 de brome. On laisse la température revenir à 20/25 °C et agite 16 heures dans ces conditions. On verse dans 200 cm3 d'eau glacée, décante, lave avec 200 cm3 d'une solution saturée de chlorure de sodium à 10% de carbonate de sodium puis avec 200 cm3 d'une solution saturée de chlorure de sodium, sèche, filtre et évapore à sec sous pression réduite. On obtient 168 g de produit recherché utilisé tel quel.

| Spectre IR : | |
|---|---|
| -C=O | 1748 $cm^{-1}$<br>1722 $cm^{-1}$ |
| Produit secondaire | 1659 $cm^{-1}$<br>1632 $cm^{-1}$ |

**Préparation 2 : 2-(méthylthio) 4-thiazole acétate de méthyle.**

**Stade A :** Acide carbamodithioïque, sel de monoammonium.

On fait circuler un courant d'ammoniac pendant 2 heures dans 60 cm3 de sulfure de carbone à +10°C. On essore et lave avec du pentane à +5°C. On obtient 75,4 g de produit attendu. F = 170°C.

Spectre IR :

Absorptions fortes région NH-OH

Absorptions région N-C=S + $NH_2$ : 1638 $cm^{-1}$, 1598 $cm^{-1}$, 1576 $cm^{-1}$

**Stade B :** 2-mercapto 4-thiazole acétate de méthyle.

A une solution de 60,6 g du produit obtenu au stade A dans 300 cm3 d'eau, on ajoute en 10 minutes 110 g de 4-bromo 3-oxo butyrate de méthyle (préparation A), on agite 16 heures à 20/25°C puis 2 heures au reflux. On refroidit à 20°C, extrait avec du chlorure de méthylène, évapore à sec et obtient 86,5 g d'huile résiduelle que l'on triture dans l'éther éthylique. On recueille 22,2 g de produit attendu. F = 114°C.

Spectre IR :

| | |
|---|---|
| C=O éther éthylique | 1741 $cm^{-1}$<br>1436 $cm^{-1}$ |
| NH + associés | : 3375 $cm^{-1}$ |
| C=C | : 1596 $cm^{-1}$ |

**Stade C :** 2-méthylthio 4-thiazole acétate de méthyle.

A une solution de 6,55 g de méthylate de sodium dans 250 cm3 de méthanol, on ajoute une suspension de 22 g de produit obtenu au stade B dans 100 cm3 de méthanol. On agite 30 minutes, ajoute 7,2 cm3 d'iodure de méthyle et chauffe 3 heures à 40°C. On évapore à sec sous pression réduite et reprend le résidu dans 300 cm3 d'eau et 300 cm3 d'éther, on agite, décante et réextrait avec 300 cm3 d'éther, sèche et amène à sec sous pression réduite. On obtient 24 g de produit attendu.

**Exemple 3 : 2-(2-méthylthio 4-thiazolyl) 3-méthoxy 2-propénoate de méthyle (isomère E) et (isomère Z).**

**Stade A :** 2-(2-méthylthio 4-thiazolyl) 3-hydroxy 2-propénoate de méthyle.

A un mélange de 1,92 g d'hydrure de sodium (à 50% dans l'huile) et 40 cm3 de tétrahydrofuranne, on ajoute une solution de 4 g de produit obtenu au stade C de la préparation 2 avec 25 cm3 de formiate

d'éthyle dans 40 cm3 de tétrahydrofuranne, on agite pendant 2 heures 30 minutes et verse dans 200 cm3 d'acide chlorhydrique 2N, agite puis extrait avec 3 fois 200 cm3 de chlorure de méthylène, sèche, filtre et évapore à sec sous pression réduite. On obtient 9,15 g de produit attendu utilisé tel quel.

**Spectre IR :**

Aborsption générale type OH

C=O : 1700 cm-1

Région C=C 1616 $cm^{-1}$

C=N 1519 $cm^{-1}$

**Stade B :** 2-(2-méthylthio 4-thiazolyl) 3-méthoxy 2-propénoate de méthyle (isomère E) et (isomère Z).

A un mélange de 2 g d'hydrure de sodium (à 50% dans l'huile) et 180 cm3 de tétrahydrofuranne, on ajoute 9,5 g du produit obtenu comme au stade A dans 90 cm3 de tétrahydrofuranne. On agite pendant 5 heures et ajoute 25,5 cm3 d'iodure de méthyle. On agite 16 heures à 20/25 °C et verse dans 1 litre d'eau, extrait avec du chlorure de méthylène, sèche, filtre et évapore à sec sous pression réduite. On chromatographie le résidu (11,5 g) sur silice (éluant : hexane-acétate d'éthyle 7-3). On obtient 3,11 g d'isomère Z, F < 50 °C et 980 mg d'isomère E.

**Isomère Z :**

**Analyse** : $C_9H_{11}NO_3S_2$

| | | | | |
|---|---|---|---|---|
| Calculé : | C% 44,06 | H% 4,52 | N% 5,71 | S% 26,14 |
| Trouvé : | 44,1 | 4,2 | 5,7 | 25,9 |

**Isomère E :**

| | | | | |
|---|---|---|---|---|
| Calculé : | C% 44,06 | H% 4,52 | N% 5,71 | S% 26,14 |
| Trouvé : | 44,3 | 4,5 | 5,8 | 25,8 |

**Exemple 4 : 2-(2-méthylthio 4-thiazolyl) 3-(2-méthoxyéthoxy) méthyloxy propénoate de méthyle (isomère Z) et (isomère E).**

On opère comme à l'exemple 2 à partir de 22,9 g de produit formylé obtenu au stade A de l'exemple 3 en utilisant 11,8 cm3 de chlorure de méthoxyéthoxyméthyle. On obtient après chromatographie (éluant : hexane-acétate d'éthyle 7-3) 18,76 g d'isomère Z et 5,8 g d'isomère E.

**Analyse** : $C_{12}H_{17}NO_5S_2$

| | | | | |
|---|---|---|---|---|
| Calculé : | C% 45,12 | H% 5,36 | N% 4,38 | S% 20,08 |
| **Isomère Z :** | | | | |
| Trouvé : | 45,0 | 5,3 | 4,2 | 20,1 |
| **Isomère E :** | | | | |
| Trouvé : | 45,1 | 5,5 | 4,3 | 19,8 |

**Préparation 3 : (E) [méthyl [1-oxo 3-(4-thiazolyl) 2-propényl]] amino] acétate de méthyle.**

**Stade A :** Méthanethioamide.

A une solution de 150 g de formamide dans 150 cm3 de tétrahydrofuranne, on ajoute à 25/30 °C, 165 g de pentasulfure de phosphore, on agite 2 heures à 25 °C, filtre et évapore le solvant sous pression réduite. On reprend avec 500 cm3 d'éther, filtre l'insoluble et évapore à sec. On reprend avec 400 cm3 d'acétate d'éthyle, traite avec du charbon actif, filtre et glace la solution à -60 °C. On essore le produit cristallisé. On obtient 103 g de produit recherché que l'on utilise tel quel pour le stade suivant. F = 20-25 °C.

**Stade B :** 4-thiazole carboxylate d'éthyle.

On opère comme au stade B de la préparation 1 à partir de 18,04 g de thioformamide obtenu au stade A en utilisant 58,5 g de bromopyruvate d'éthyle. On obtient après chromatographie sur silice (éluant : chlorure de méthylène-éther 95-5) 25,9 g de produit attendu isolé de l'éther de pétrole (Eb : 40-70 °C). F = 52 °C.

| Analyse : $C_6H_7O_2NS$ | | | | | | | | |
|---|---|---|---|---|---|---|---|---|
| Calculé<br>Trouvé | C% | 45,8<br>45,8 | H% | 4,54<br>4,5 | N% | 8,9<br>8,9 | S% | 20,4<br>20,7 |

| Spectre IR ($CHCl_3$) : | |
|---|---|
| C=O<br>hétérocycle | 1724 $cm^{-1}$<br>1501, 1486 $cm^{-1}$ |

**Stade C :** 4-hydroxyméthylthiazole.

On opère comme au stade C de la préparation 1 à partir de 35 g de thiazol-4-yl carboxylate d'éthyle. On obtient 18,3 g de produit recherché utilisé tel quel pour le stade suivant.

**Stade D :** 4-formylthiazole.

On opère comme au stade D de la préparation 1 à partir de 18,3 g de 4-hydroxyméthylthiazole obtenu au stade C en utilisant 87 g de bioxyde de manganèse. Après chromatographie sur silice (éluant : hexane-acétate d'éthyle 1-1), on obtient 13,9 g de produit recherché.

Spectre IR ($CHCl_3$ sur Nicolet) :
peu ou pas d'OH

| | |
|---|---|
| >C=O | : 1705 $cm^{-1}$ |
| région aldéhyde CH= | 2750 $cm^{-1}$ |
| | 2840 $cm^{-1}$ |
| système conjugué | 1570 $cm^{-1}$ |
| | 1485 $cm^{-1}$ |

**Stade E :** Acide (E)-3-(4-thiazolyl) propénoïque.

On opère comme au stade E de la préparation 1 à partir de 5,65 g de 4-formylthiazole obtenu au stade D. On obtient 7,2 g de produit recherché. F = 170 °C.

| Spectre IR (Nujol sur Nicolet) : | |
|---|---|
| C = O complexe<br>-C = C- | 1691 $cm^{-1}$<br>1636 $cm^{-1}$ |
| système conjugué | 1529 $cm^{-1}$<br>1489 $cm^{-1}$ |
| HC = CH | 978 $cm^{-1}$ |

**Stade F :** (E) [méthyl [1-oxo 3-(4-thiazolyl) 2-propényl] amino] acétate de méthyle.

On opère comme au stade F de la préparation 1 à partir de 3,9 g de produit obtenu au stade E. Après chromatographie sur silice (éluant : hexane-acétate d'éthyle 3-7), on obtient 4,7 g de produit recherché.

| Spectre IR ($CHCl_3$ sur Nicolet) : | |
|---|---|
| C = O<br>amide<br>>C = C<br>thiazole | 1751 $cm^{-1}$ C = O<br>1654 $cm^{-1}$<br>1611 $cm^{-1}$<br>1494 $cm^{-1}$ |

**Exemple 5 : (E,Z) 3-méthoxy [2-méthyl [1-oxo 3-(4-thiazolyl) 2-propényl] amino] 2-propénoate de méthyle.**

**A - Formylation :**

On opère comme au stade A de l'exemple 3 à partir de 4,08 g du produit obtenu au stade F de la préparation 3.

**B - Méthylation :**

On opère comme au stade B de l'exemple 1 à partir de 3,12 g de produit obtenu ci-dessus. On obtient 3,3 g de produit après chromatographie sur silice (éluant : hexane-acétate d'éthyle 3-7). Le produit est dissous dans le chlorure de méthylène, on filtre l'insoluble et évapore à sec sous pression réduite. On recueille 2,0 g de produit recherché. F = 108 °C.

| Analyse : $C_{12}H_{14}N_2O_4S$ | | | | | | |
|---|---|---|---|---|---|---|
| Calculé<br>Trouvé | C% | 51,05<br>51,0 | H% | 5,00<br>4,8 | N% | 9,92<br>9,9 |

**Spectre IR ($CHCl_3$) :**

| | |
|---|---|
| C=O ester | : 1717 $cm^{-1}$ |
| ester méthylique | : 1439 cm-1 |
| C=O amide | : 1652 $cm^{-1}$ |
| C=C | : 1617 $cm^{-1}$ |
| thiazole | 1490 $cm^{-1}$<br>3115 $cm^{-1}$ |

**Exemple 6 : (E,Z) 3-[[(2-méthoxy) éthoxy] méthoxy] 2-[méthyl [1-oxo 3-(4- thiazolyl) 2-propényl] amino] 2-propénoate de méthyle.**

**A - Formylation :**

On opère comme en A de l'exemple 5 à partir de 3,8 g de produit obtenu au stade F de la préparation 3. On obtient 1,9 g de produit formylé utilisé tel quel.

**B - Méthylation :**

On opère comme a l'exemple 2 à partir de 1,9 g du produit formylé ci-dessus en utilisant 1,1 cm3 de chlorure de méthoxyéthoxyméthyle. Après chromatographie sur silice (éluant : acétate d'éthyle), on obtient 1,68 g de produit recherché. F = 86 ° C.

| Spectre IR ($CHCl_3$ sur PE 580) : | |
|---|---|
| C = O ester | 1718 $cm^{-1}$ |
| C = O amide | 1654 $cm^{-1}$ |
| -C = C- | 1617 $cm^{-1}$ |
| système conjugué thiazole | 1490 $cm^{-1}$ |
| $CH_3$ de l'ester méthylique | 1439 $cm^{-1}$ |

| Analyse : $C_{15}H_{20}N_2O_6S$ | | | | | | | | |
|---|---|---|---|---|---|---|---|---|
| Calculé<br>Trouvé | C% | 50,55<br>50,3 | H% | 5,66<br>5,8 | N% | 7,86<br>7,9 | S% | 9,00<br>8,8 |

**Préparation 5 : (4-chlorophényl) 4-thiazole acétate de méthyle.**

**Stade A :** 4-chlorobenzène carbothioamide.

On fait barboter pendant 3 heures à 20/25 ° C un courant d'hydrogène sulfuré dans un mélange de 20 g de 4-chlorobenzonitrile, 28 cm3 de pyridine, 22 cm3 de triéthylamine. On coule dans l'eau, essore et obtient 24 g de produit attendu. F = 132 ° C.

**Spectre IR :**

| | |
|---|---|
| $=C-NH_2$ | 3488 $cm^{-1}$<br>3380 $cm^{-1}$ |
| aromatique | 1600 cm-1 |
| + $NH_2$ | 1489 $cm^{-1}$ |

**Stade B :** 2-(4-chlorophényl) 4-thiazole acétate de méthyle.

A une solution de 17,1 g de 4-chlorothiobenzamide obtenue au stade A dans 150 cm3 de méthanol, on ajoute une solution de 19,5 g de 4-bromo 3-oxo butyrate de méthyle (préparation A) dans 40 cm3 de méthanol. On chauffe au reflux pendant 2 heures et évapore le solvant sous pression réduite. On reprend avec 500 cm3 d'une solution 1M de bicarbonate de sodium et extrait avec 2 fois 500 cm3 d'éther éthylique. On évapore à sec et chromatographie le résidu (25,5 g) sur silice (éluant : hexane-acétate d'éthyle 7-3). On obtient 19,8 g de produit recherché. F = 74 ° C.

**Spectre IR :**

| | |
|---|---|
| >C=O | : 1736 $cm^{-1}$ |
| $CH_3$ de l'ester méthylique | : 1439 $cm^{-1}$ |
| hétérocycle + aromatique | 1597 $cm^{-1}$<br>1520 $cm^{-1}$<br>1499 $cm^{-1}$ |

**Exemple 7 : 2-(4-chlorophényl) alpha-(méthoxy méthylène) 4-thiazole acétate de méthyle isomères (E) + (Z).**

**Stade A :** 2-(4-chlorophényl) alpha-(hydroxy méthylène) 4-thiazole acétate de méthyle.

On opère comme au stade A de l'exemple 3 à partir de 5,35 g de produit obtenu au stade B de la préparation 5 en utilisant 25 cm3 de formiate de méthyle. On obtient 6 g de produit recherché. F = 144 °C.

**Spectre IR :**

Absorption générale type NH-OH

| | |
|---|---|
| C=O région ester conjugué | : 1700 $cm^{-1}$ |
| -C=C- | : 1617 $cm^{-1}$ |
| Système conjugué + aromatiques | 1598 $cm^{-1}$<br>1520 $cm^{-1}$<br>1501 $cm^{-1}$ |

**Stade B :** 2-(4-chlorophényl) alpha-(méthoxy méthylène 4-thiazole acétate de méthyle isomères (E) + (Z).

On opère comme au stade B de l'exemple 3 à partir de 8,4 g de produit préparé comme au stade A. On obtient, après chromatographie sur silice (éluant hexane-acétate d'éthyle 7-3), 2,72 g du produit recherché, isomère Z F = 109 °C et 0,25 g d'isomère E. On a également préparé de l'isomère E en maintenant 24 heures au reflux 2,1 g du produit isomère Z dans 22 cm3 de méthanol en présence de 210 mg d'acide paratoluènesulfonique. Après évaporation à sec et chromatographie, on obtient 0,87 g d'isomère Z et 0,89 g d'isomère E.

**Analyse** : $C_{14}H_{12}ClNO_3S$

**Isomère Z** :

| | C% | H% | Cl% | N% | S% |
|---|---|---|---|---|---|
| Calculé : | 54,28 | 3,91 | 11,44 | 4,52 | 10,35 |
| Trouvé : | 54,4 | 3,8 | 11,7 | 4,5 | 10,1 |

**Isomère E** :

| | C% | H% | Cl% | N% | S% |
|---|---|---|---|---|---|
| Trouvé: | 54,4 | 3,8 | 11,4 | 4,3 | 10,1 |

**Exemple 8 : 2-(4-chlorophényl) alpha-3-[[2-méthoxy (éthoxy)] méthoxy] 4- thiazole acétate de méthyle isomères (E) + (Z).**

On opère comme à l'exemple 2 à partir de 2,8 g du produit obtenu au stade A de l'exemple 7 en utilisant 1,13 cm3 de chlorure de méthoxyéthoxyméthyle. On obtient après chromatographie sur silice (éluant : hexane-acétate d'éthyle 7-3) 2,14 g de produit isomère Z et 0,31 g de produit isomère E. F < 50 °C.

**Analyse** : $C_{17}H_{18}ClNO_5S$

**Isomère Z** :

| | | | | | |
|---|---|---|---|---|---|
| Calculé : | C% 53,19 | H% 4,72 | Cl% 9,24 | N% 3,65 | S% 8,35 |
| Trouvé : | 53,3 | 4,7 | 9,2 | 3,6 | 8,1 |

**Isomère E** :

| | | | | | |
|---|---|---|---|---|---|
| Trouvé : | 53,1 | 4,9 | 9,0 | 3,5 | 8,0 |

**Préparation 6 : 4-(4-chlorophényl) 2-(4-cyclopropylphényl) 5-thiazol acétate de méthyle.**

On agite 16 heures au reflux un mélange de 20 g d'acide 2-(4-cyclopropylphényl) 4-(4-chlorophényl) 5-thiazolyle acétique (obtenus selon BF.2 096 994), 200 cm3 de méthanol et 0,25 cm3 d'acide sulfurique concentré. On refroidit à 0 ° C, essore et obtient 16,36 g de produit recherché. F = 98 ° C.

**Spectre IR** :

| | |
|---|---|
| >C=O région ester | : 1742 $cm^{-1}$ |
| $CH_3$ de $COOCH_3$ | : 1438 $cm^{-1}$ |
| Système conjugué | 1612 $cm^{-1}$ |
| | 1600 cm-1 |
| + | 1570 $cm^{-1}$ |
| | 1522 $cm^{-1}$ |
| aromatiques | 1488 $cm^{-1}$ |

**Exemple 9 : 4-(4-chlorophényl) 2-(4-cyclopropylphényl) alpha-(méthoxy méthylène 5-thiazole acétate de méthyle isomères E et Z.**

**Stade A** : 4-(4-chlorophényl) 2-(4-cyclopropylphényl) alpha-hydroxy méthylène 5-thiazole acétate de méthyle.

On opère comme au stade A de l'exemple 3 à partir de 9,6 g de produit obtenu à la préparation 6. On obtient 8,5 g de produit recherché. F = 174 ° C.

**Spectre IR** :

Absorptions régions NH-OH tri associés

| | |
|---|---|
| >C=O | : 1714 $cm^{-1}$ |
| >C=O<br>-C=C- | 1669 $cm^{-1}$ |
| Système conjugué | 1640 $cm^{-1}$ |
| | 1628 $cm^{-1}$ |
| + | 1608 $cm^{-1}$ |
| | 1571 $cm^{-1}$ |
| | 1523 $cm^{-1}$ |
| aromatiques | 1483 $cm^{-1}$ |

**Stade B** : 4-(4-chlorophényl) 2-(4-cyclopropylphényl) alpha-(méthoxy méthylène 5-thiazole acétate de méthyle isomères E et Z.

On opère comme au stade B de l'exemple 3 à partir de 4,11 g de produit obtenu au stade A. On obtient 0,94 g de produit attendu isomère Z, F = 114°C et 1,82 g de produit isomère E, F = 153°C.

**Analyse** : $C_{23}H_{20}ClNO_3S$

| | C% | H% | Cl% | N% | S% |
|---|---|---|---|---|---|
| **Isomère Z** : | | | | | |
| Calculé : | 64,85 | 4,73 | 8,32 | 3,29 | 7,53 |
| Trouvé : | 64,8 | 4,7 | 8,1 | 3,3 | 7,5 |
| **Isomère E** : | | | | | |
| Trouvé : | 64,8 | 4,7 | 8,0 | 3,2 | 7,4 |

**Exemple 10 : 4-(4-chlorophényl) 2-(4-cyclopropylphényl) alpha-[[(2-méthoxy éthoxy) méthoxy] méthylène] 5-thiazole acétate de méthyle isomères E et Z.**

On opère comme à l'exemple 2 à partir de 4,11 g de produit obtenu au stade A de l'exemple 9 en utilisant 1,2 cm3 de chlorure de méthoxyéthoxy- méthyle. On obtient, après chromatographie sur silice (éluant : hexane-acétate d'éthyle 7-3), 0,73 g de produit attendu isomère Z et 4 g d'isomère E. F = 82°C.

**Analyse** : $C_{26}H_{26}ClNO_5S$

| | C% | H% | Cl% | N% | S% |
|---|---|---|---|---|---|
| **Isomère Z** : | | | | | |
| Calculé : | 62,45 | 5,24 | 7,09 | 2,8 | 6,41 |
| Trouvé : | 62,3 | 5,2 | 7,2 | 2,7 | 6,3 |
| **Isomère E** : | | | | | |
| Trouvé : | 62,3 | 5,2 | 7,1 | 2,7 | 6,3 |

**Préparation 7 : (2-méthylthio 4-méthyl 5-thiazolyl) acétate de méthyle.**

**Stade A** : (2-thioxo 4-méthyl 5-thiazolyl) acétate de méthyle.

On agite au reflux pendant 1 heure 18,9 g d'acide 2-mercapto 4-méthyl 5-thiazole acétique (obtenu selon Yujiro Haro et coll, Pharm. Bull 2, 156 (1954)), 190 cm3 de méthanol et 0,2 cm3 d'acide sulfurique concentré. On distille à sec, triture le résidu à l'éther (__ 100 cm3), essore et obtient 19,5 g de produit recherché. F = 138°C.

**Spectre IR** :

| | |
|---|---|
| C=O | : 1742 $cm^{-1}$ |
| -C=C-X | 1628 $cm^{-1}$ |
| | 1480 $cm^{-1}$ |
| NH + associé | : 3390 $cm^{-1}$ |

**Stade B** : (2-méthylthio 4-méthyl 5-thiazolyl) acétate de méthyle.

On agite pendant 30 minutes une suspension de 19 g du produit obtenu au stade A ci-dessus, 70 cm3 d'eau et 3,75 g de soude pastilles, et ajoute ensuite 9,5 cm3 de sulfate diméthylique. On agite 5 heures à 20/25°C, filtre, extrait avec 2 fois 200 cm3 de chlorure de méthylène, évapore à sec sous pression réduite et chromatographie le résidu sur silice (éluant : hexane-acétate d'éthyle 4-6). On obtient 13,4 g de produit

recherché.

**Spectre IR :**

Absence de NH
ester méthylique : 1738 $cm^{-1}$, 1437 $cm^{-1}$
bande de système conjugué : 1555 $cm^{-1}$

**Exemple 11 : 2-(2-méthylthio 4-méthyl 5-thiazolyl) 3-méthoxy 2-propénoate de méthyle (isomères E et Z).**

**Stade A :** 2-(2-méthylthio 4-méthyl 5-thiazolyl) 3-hydroxy 2-propénoate de méthyle.

On opère comme au stade A de l'exemple 3 à partir de 13,4 g du produit obtenu au stade B de la préparation 7. On obtient après chromatographie sur silice (éluant : hexane-acétate d'éthyle 7-3), 4,6 g du produit recherché. F = 107°C.

**Spectre IR :**

| | |
|---|---|
| absorption région NH-OH | : 3480 $cm^{-1}$ |
| -C(=O)- complexe ester conjugué | 1700 $cm^{-1}$ |
| | 1674 $cm^{-1}$ |
| Région C=C + hétérocycle | 1643 $cm^{-1}$ |
| | 1622 $cm^{-1}$ |
| | 1610 $cm^{-1}$ |
| | 1546 $cm^{-1}$ |
| Méthyl de l'ester | : 1440 $cm^{-1}$ |

**Stade B :** 2-(2-méthylthio 4-méthyl 5-thiazolyl) 3-méthoxy 2-propénoate de méthyle (isomères E et Z).

On opère comme au stade B de l'exemple 3 à partir de 4 g du produit obtenu au stade A ci-dessus. On obtient, après chromatographie sur silice (éluant : hexane-acétate d'éthyle 4-6), 0,5 g de produit recherché isomère Z et 0,51 g d'isomère E.

**Analyse** : $C_{10}H_{13}NO_3S_2$

**Isomère Z** :

| | C% | H% | N% | S% |
|---|---|---|---|---|
| Calculé : | 46,31 | 5,05 | 5,40 | 27,72 |
| Trouvé : | 46,1 | 5,1 | 5,3 | 24,4 |

**Isomère E** :

| | C% | H% | N% | S% |
|---|---|---|---|---|
| Trouvé : | 46,1 | 5,1 | 5,3 | 24,4 |

**Exemple 12 : 2-(2-méthylthio 4-méthyl 5-thiazolyl) 3-(2-méthoxyéthoxy) méthoxy propénoate de méthyle (isomères Z et E).**

On opère comme à l'exemple 2 à partir de 4,94 g du produit obtenu au stade A de l'exemple 11 et on obtient, après chromatographie sur silice (éluant : hexane-acétate d'éthyle 4-6), 1,5 g de produit recherché isomère Z et 4,44 g d'isomère E.

**Analyse** : $C_{13}H_{19}NO_5S_2$

**Isomère Z** :

| | | | | |
|---|---|---|---|---|
| Calculé : | C% 46,83 | H% 5,74 | N% 4,20 | S% 19,23 |
| Trouvé : | 46,5 | 5,8 | 4,1 | 19,4 |

**Isomère E** :

| | | | | |
|---|---|---|---|---|
| Trouvé : | 46,7 | 5,9 | 4,0 | 19,2 |

**Préparation 4 : [2'-éthyl (2,4'-bithiazol) 4-yl] carbaldéhyde.**

**Stade A :** Thiopropionamide.

A un mélange de 7,31 g de propionamide dans 350 cm3 de tétrahydrofuranne, on ajoute à 20/25 ° C par portions 20,22 g de réactif de Lawesson, on agite à 20/25 ° C pendant 16 heures, évapore à sec sous pression réduite. On chromatographie le résidu sur silice (éluant : hexane-acétate d'éthyl 7-3), et obtient 8 g de produit attendu. F < 50 ° C.

**Spectre IR :**

$=C-NH_2$ : 3492 $cm^{-1}$, 3448 $cm^{-1}$, 3380 $cm^{-1}$

$NH_2$ : 1607 $cm^{-1}$

**Stade B :** 4-éthoxycarbonyl 2-éthylthiazole.

On opère comme au stade B de la préparation 1 à partir de 33 g de produit obtenu comme au stade A ci-dessus. On obtient 55 g de produit recherché. F < 50 ° C.

**Spectre IR :**

C=O : 1721 $cm^{-1}$

Système conjugué : 1603 $cm^{-1}$, 1503 $cm^{-1}$, 1488 $cm^{-1}$

**Stade C :** 4-carbamoyl 2-éthylthiazole.

Dans un appareil permettant de travailler sous pression, on mélange à -70 ° C 24 g du produit obtenu au stade B, 750 cm3 de méthanol et 84 cm3 d'ammoniac liquéfié. On laisse la température revenir à 20/25 ° C. on agite pendant 16 heures puis évapore le solvant. On chromatographie sur silice (éluant : hexane-acétate d'éthyle 1-1). On recueille 17 g du produit recherché. F = 122 ° C.

Spectre IR :

| | |
|---|---|
| $=C-NH_2$ | 3519 $cm^{-1}$<br>3401 $cm^{-1}$<br>3471 $cm^{-1}$ |
| C=O | 1682 $cm^{-1}$ |
| Système conjugué et $NH_2$ | 1573 $cm^{-1}$<br>1515 $cm^{-1}$<br>1493 $cm^{-1}$ |

**Stade D :** 2-éthyl 4-thiocarbamoylthiazole.

On agite pendant 1 heure au reflux 2 g du produit obtenu au stade C ci-dessus, 100 cm3 de toluène et 2,59 g de réactif de Lawesson. On évapore à sec et chromatographie le résidu sur silice (éluant : hexane-acétate d'éthyle 7-3). On recueille 2,2 g de produit attendu. F = 140 ° C.

| Spectre IR : | |
|---|---|
| $=C-NH_2$ | 3484 $cm^{-1}$<br>3350 $cm^{-1}$ |
| $NH_2$ déf. | 1584 $cm^{-1}$ |
| Système conjugué | 1506 $cm^{-1}$<br>1490 $cm^{-1}$ |

**Stade E :** 4-éthoxycarbonyl 2'-éthyl 2,4'-bithiazole.

On opère comme au stade B de la préparation 1 à partir de 2 g du produit obtenu au stade D ci-dessus. On recueille, après chromatographie sur silice (éluant : hexane-acétate d'éthyle 7-3), 2,52 g du produit recherché. F < 50 ° C.

| Spectre IR ($CHCl_3$ sur PE 580) : | |
|---|---|
| >C=O<br>C=C<br>C=N | 1722 $cm^{-1}$<br>1543 $cm^{-1}$ |

**Stade F :** [2'-éthyl (2,4'-bithiazol) 4-yl] méthanol.

On opère comme au stade C de la préparation 1 à partir de 2,5 g du produit obtenu au stade E ci-dessus. On obtient, après chromatographie sur silice (éluant : hexane-acétate d'éthyle 1-1), 2 g de produit recherché. F = 90 ° C.

| Spectre IR ($CHCl_3$ sur PE 580) : | |
|---|---|
| -OH | 3609 $cm^{-1}$ |
| Système conjugué | 1538 $cm^{-1}$<br>1497 $cm^{-1}$ |

**Stade G :** [2'-éthyl (2,4'-bithiazol) 4-yl] carbaldéhyde.

On opère comme au stade D de la préparation 1 à partir de 1,25 g du produit obtenu au stade F ci-dessus. On obtient après chromatographie sur silice (éluant : hexane-acétate d'éthyle 1-1), 0,94 g de produit recherché. F = 98 °C.

**Spectre IR** ($CHCl_3$ sur PE 580) :

Peu ou pas d'OH

| | |
|---|---|
| C=O aldéhyde | 1700 $cm^{-1}$ |
| =C-H | 2760 $cm^{-1}$ |
| hétéroaromatique | 1542 $cm^{-1}$ |

**Préparation 8 : [N-[3-[2'-éthyl (2,4'-bithiazol)-4-yl] 1-oxo 2-(E)-propényl] méthylamino] acétate de mèthyle**.

**Stade A :** Acide (E) [2'-éthyl (2,4'-bithiazol) 4-yl] propénoïque.

On opère comme au stade E de la préparation 1 à partir de 4 g de produit obtenu au stade G de la préparation 4. On obtient 4,4 g de produit recherché. F = 208 °C.

**Stade B :** [N-[3-[2'-éthyl (2,4'-bithiazol)-4-yl] 1-oxo 2-(E)-propényl] méthylamino] acétate de méthyle.

A un mélange de 1,71 g de méthylaminoacétate de méthyle, 4,4 g d'acide préparé au stade A, 0,4 g de diméthylaminopyridine et 40 cm3 de chlorure de méthylène, on ajoute à 20/25 °C, 3,82 g de dicyclohexylcarbodiimide en solution dans 40 cm3 de chlorure de méthylène. On agite 16 heures, essore l'insoluble, évapore à sec et chromatographie sur silice (éluant : hexane-acétate d'éthyle 1-1). On obtient 3,1 g de produit recherché. F = 189 °C.

| Spectre IR : $CHCl_3$ | |
|---|---|
| C=O | 1751 $cm^{-1}$<br>1653 $cm^{-1}$ |
| C=C | 1609 $cm^{-1}$<br>1522 $cm^{-1}$ |
| hétérocycle<br>-CH=CH-trans | 1491 $cm^{-1}$<br>974 $cm^{-1}$ |

**Exemple 13 : 2-[N-[3-[2'-éthyl (2,4'-bithiazol) 4-yl] 1-oxo 2-(E)-propényl] méthylamino] 3-(Z)-méthoxy propénoate de méthyle.**

A un mélange de 0,82 g d'hydrure de sodium à 50% dans l'huile et 60 cm3 de diméthylformamide, on ajoute une solution de 3 g du produit obtenu au stade B de la préparation 8 avec 10,5 cm3 de formiate de méthyle dans 50 cm3 de diméthylformamide. On agite à 20/25 °C jusqu'au démarrage de la réaction (dégagement gazeux abondant après 5 heures), on agite encore 1 heure ensuite et ajoute 5 cm3 de formiate de méthyle. On agite pendant 16 heures et ajoute 16 cm3 d'iodure de méthyle. On agite pendant 2 heures à 20/25 °C puis verse sur une solution d'acide chlorhydrique 2N et glace. On extrait avec de l'éther isopropylique et évapore à sec sous pression réduite. On chromatographie le résidu sur silice (éluant : hexane-acétate d'éthyle 2-8). On recueille 1,5 g de produit recherché.

| Analyse : $C_{17}H_{19}N_3O_4S_2$ | | | | | | | | |
|---|---|---|---|---|---|---|---|---|
| Calculé<br>Trouvé | C% | 51,89<br>51,6 | H% | 4,87<br>4,8 | N% | 10,68<br>10,5 | S% | 16,30<br>16,1 |

**Préparation 9 : Acide [2'-éthyl (2,4'-bithiazol) 4-yl] 2-(E)-fluoropropénoïque.**

**Stade A :** [2'-éthyl (2,4'-bithiazol) 4-yl] 2-fluoro (E) propénoate de méthyle.

A une solution de 5,56 g de bromure de lithium dans 55 cm3 de tétrahydrofuranne refroidi à -30 ° C, on ajoute 4,5 cm3 de diisopropylamine. On refroidit à -60 ° C et ajoute, sans dépasser -55 ° C, 4 g du produit obtenu au stade G de la préparation 4 et 5,18 g de diéthylphosphonofluoroéthanoate de méthyle dans 50 cm3 de tétrahydrofuranne. On agite 1 heure à -60 ° C puis verse sur une solution d'acide chlorhydrique 2N. On essore et dissout le produit obtenu dans le chloroforme, sèche, filtre et distille à sec. On obtient 5,1 g de produit recherché. F = 140 ° C.

**Spectre IR** ($CHCl_3$ sur PE 580) :

| | | |
|---|---|---|
| C=O | : | 1731 $cm^{-1}$ |
| CO $OCH_3$ avec $CH_3$ | : | 1438 $cm^{-1}$ |
| C=C | | 1668 $cm^{-1}$ |
| C=N | | 1652 $cm^{-1}$ |
| hétéroaromatique | | 1540 $cm^{-1}$<br>1500 $cm^{-1}$ |

**Stade B :** Acide [2'-éthyl (2,4'-bithiazol) 4-yl] 2-(E)-fluoropropénoïque.

On agite pendant 16 heures à 20/25 ° C 5,1 g du produit obtenu au stade A ci-dessus, 100 cm3 de méthanol et 20 cm3 d'une solution de soude N. On lave avec du chlorure de méthylène, acidifie la phase aqueuse puis extrait avec du chlorure de méthylène, sèche, filtre et évapore à sec. On obtient 4,8 g de produit recherché. F = 153 ° C.

| Spectre IR : | |
|---|---|
| OH<br>C = O complexe<br>C = C<br>C = N | région 3000 à 2000 $cm^{-1}$<br>1730 $cm^{-1}$<br>1642 $cm^{-1}$<br>1635 $cm^{-1}$ |
| hétéroaromatique | |

**Exemple 14 : 2-[N-[3-[2'-éthyl (2,4'-bithiazol) 4-yl] 1-oxo 2-(Z)-fluoro propényl] méthylamino] 3-méthoxy (Z) propénoate de méthyle.**

**Stade A :** 2-[N-[3-[2'-éthyl (2,4'-bithiazol) 4-yl] 2-(Z)-fluoro 1-oxo propényl] méthylamino] 3,3-diméthoxy propanoate de méthyle.

On chauffe au reflux pendant 1 heure 30 minutes, 4,7 g du produit obtenu au stade B de la préparation 9 et 50 cm3 de chlorure de thionyle. On ramène à 20/25 ° C et évapore le chlorure de thionyle en excès. On reprend par 50 cm3 de chloroforme et ajoute cette solution sous agitation à 20/25 ° C dans le mélange de 7,09 g de méthylaminodiméthoxy propanoate de méthyle (EP 0 178 808) et 100 cm3 de chloroforme. On agite 30 minutes, évapore à sec et chromatographie le résidu sur silice (éluant : hexane-acétate d'éthyle 1-1). On obtient 3,2 g du produit recherché.

| Spectre IR ($CHCl_3$ sur PE 580) : | |
|---|---|
| C=O complexe<br>C-OMe<br>O | 1740 $cm^{-1}$<br>1439 $cm^{-1}$ |
| C=O + C=C | 1660 $cm^{-1}$<br>1649 $cm^{-1}$<br>1538 $cm^{-1}$ |
| Système conjugué<br>$CH_3$ | 1498 $cm^{-1}$<br>2840 $cm^{-1}$ |

**Stade B :** 2-[N-[3-[2'-éthyl (2,4'-bithiazol) 4-yl] 1-oxo 2-(Z)-fluoropropényl] méthylamino] 3-méthoxy (E) propénoate de méthyle.

On chauffe au reflux pendant 1 heure 30 minutes en éliminant le méthanol formé un mélange de 3,2 g du produit obtenu au stade A ci-dessus et 50 cm3 de toluène avec 30 mg d'acide paratoluènesulfonique. On verse sur de l'eau, extrait avec du chlorure de méthylène, sèche, filtre et amène à sec. On chromatographie le résidu sur silice (éluant : hexane-acétate d'éthyle 1-1) et obtient 1,76 g de produit recherché. F = 99 ° C.

| Analyse : $C_{17}H_{18}N_3O_4S_2F$ | | | | | | | | | | |
|---|---|---|---|---|---|---|---|---|---|---|
| Calculé<br>Trouvé | C% | 49,62<br>49,8 | H% | 4,41<br>4,4 | N% | 10,21<br>9,9 | S% | 15,58<br>15,4 | F% | 4,62<br>4,5 |

**Préparation 10 : Acide 3-(4-thiazolyl) 2-fluoro 2-(E)-propénoïque.**

**Stade A :** 2-fluoro 3-(4-thiazolyl) 2-(E)-propénoate de méthyle.

On opère comme au stade A de la préparation 9 à partir de 6 g du produit obtenu au stade D de la préparation 3 en utilisant 15,3 g de diéthylphosphono fluoro éthanoate de méthyle. On obtient, après chromatographie sur silice (éluant : hexane-acétate d'éthyle 9-1), 6,4 g du produit recherché utilisé tel quel pour le stade suivant.

| Spectre IR : | |
|---|---|
| >C=O<br>-C=C-<br>+<br>Système conjugué<br>$CH_3$ du $COOCH_3$ | 1734 $cm^{-1}$<br>1438 $cm^{-1}$<br><br>1656 $cm^{-1}$<br>1440 $cm^{-1}$ |

**Stade B :** Acide 3-(4-thiazolyl) 2-fluoro 2-(E)-propénoïque.

On opère comme au stade B de la préparation 9 à partir de 6,4 g du produit obtenu au stade A ci-dessus. On obtient 5,2 g de produit recherché.

| Spectre IR (Nujol sur Nicolet) : Absorption générale OH/NH | |
|---|---|
| ⟩C=O complexe<br>-C=C- | 1712 $cm^{-1}$<br>1641 $cm^{-1}$ |
| Système conjugué | 1575 $cm^{-1}$<br>1551 $cm^{-1}$ |

**Exemple 15 : (Z,Z) 2-[(2-fluoro 1-oxo 3-(4-thiazolyl) 2-propényl) N-méthylamino] 3-méthoxy 2-propénoate de méthyle.**

**Stade A :** 2-[N-[3-(4-thiazolyl) 1-oxo 2-(Z)-fluoropropényl] méthylamino] 3,3-diméthoxy propanoate de méthyle.

**A - Préparation du chlorure d'acide.**

On agite au reflux pendant 2 heures 30 minutes 5,2 g du produit obtenu au stade B de la préparation 10 et 20 cm3 de chlorure de thionyle. On évapore à sec, entraîne avec de l'hexane et obtient 6,6 g de chlorure d'acide.

**B - Condensation.**

On agite à température ambiante 15 g de chlorhydrate de méthylamino diméthoxy propanoate de méthyle, 150 cm3 de chloroforme et 13,1 g de bicarbonate de sodium. A la fin du dégagement gazeux, on ajoute du sulfate de sodium et filtre. A la solution obtenue, on ajoute 13,1 g de bicarbonate de sodium puis, en 30 minutes, une solution de 6,6 g de chlorure d'acide obtenu en A dans 100 cm3 de chloroforme. On agite 16 heures à température ambiante puis coule sur de l'acide chlorhydrique 2N. On décante et réextrait avec 5 fois 300 cm3 de chloroforme, sèche, filtre et évapore à sec. On chromatographie le résidu sur silice (éluant : hexane-acétate d'éthyle 4-6) et obtient 5,9 g de produit recherché.

| Spectre IR ($CNCl_3$ sur PE 580) : | |
|---|---|
| C=O type -C-$OCH_3$ non conjugué<br>amide tertiaire + -C=C-<br>$CH_3$ | 1739 $cm^{-1}$<br>1639 $cm^{-1}$<br>1439 $cm^{-1}$ |

**Stade B :** (Z,Z) 2-[(2-fluoro 1-oxo 3-(4-thiazolyl) 2-propényl) N-méthyl amino] 3-méthoxy 2-propénoate de méthyle.

On agite pendant 5 heures 30 minutes à 130 °C, en distillant le méthanol formé, un mélange de 5,9 g du produit obtenu au stade A ci-dessus avec 60 cm3 de toluène et 60 mg d'acide paratoluènesulfonique. Après 1 heure, on ajoute 200 mg d'acide paratoluènesulfonique. Après 16 heures de repos, on ajoute 500 mg d'acide paratoluènesulfonique et on agite 1 heure 30 minutes au reflux en distillant le méthanol formé. On refroidit, lave à l'eau et évapore à sec sous pression réduite. On chromatographie sur silice (éluant : chlorure de méthylène-acétate d'éthyle 1-1) et obtient 1,55 g du produit attendu que l'on dissout dans le chlorure de méthylène, filtre et amène à sec sous pression réduite. On recueille 1,1 g de produit recherché.

Spectre IR ($CHCl_3$ sur PE 580) :

C=O région ester conjugué : 1717 $cm^{-1}$

$CH_3$ du $COOCH_3$ : 1439 $cm^{-1}$

-C=C- + amide tertiaire { 1672 $cm^{-1}$, 1653 $cm^{-1}$, 1630 $cm^{-1}$

| Analyse : $C_{12}H_{13}FN_2O_4S$ | | | | | | | | | |
|---|---|---|---|---|---|---|---|---|---|
| Calculé<br>Trouvé | C% | 47,99<br>47,7 | H% | 4,36<br>4,3 | F% | 6,33<br>6,0 | N% | 9,33<br>9,1 | S% | 10,68<br>10,4 |

**Exemple 16 : (E,E) alpha-(méthoxy méthylène) 2-[2-(4-thiazolyl) éthényl] benzène acétate de méthyle.**

A une suspension de 0,59 g d'hydrure de sodium (à 50% dans l'huile) et 20 cm3 de tétrahydrofuranne, on ajoute 3,87 g d'alpha [2-(1,3-diméthoxy 1-oxo 2-propényl phényl] méthyl phosphonate de diméthyle (brevet allemand 3 620 860) en solution dans 10 cm3 de tétrahydrofuranne. On agite 1 heure puis refroidit à +10°C et ajoute 1,39 g de produit obtenu au stade D de la préparation 3 en solution dans 10 cm3 de tétrahydrofuranne. On agite pendant 16 heures à 20/25°C. On coule dans 100 cm3 d'eau et extrait avec 3 fois 100 cm3 d'éther. On évapore à sec sous pression réduite, chromatographie le résidu sur silice (éluant : hexane-acétate d'éthyle 4-6) et obtient 1,12 g de produit recherché. F = 106°C.

| Analyse : $C_{16}H_{15}NO_3S$ | | | | | | | | |
|---|---|---|---|---|---|---|---|---|
| Calculé<br>Trouvé | C% | 63,76<br>63,6 | H% | 5,01<br>5,0 | N% | 4,65<br>4,8 | S% | 10,64<br>10,6 |

**Exemple 17 : 2-(E)-[[[2'-éthyl (2,4'-bithiazol) 4-yl] éthényl] 2-phényl] 3-méthoxy 2-(E)-propénoate de méthyle.**

On opère comme à l'exemple 16 à partir de 2,24 g du produit obtenu au stade G de la préparation 4 en utilisant 3,14 g d'alpha [2-(1,3-diméthoxy 1-oxo 2-propényl)phényl] méthyl phsophonate de diméthyle (brevet allemand 3 620 860). On obtient, après chromatographie sur silice (éluant : hexane-acétate d'éthyle 7-3), 1,8 g de produit recherché. F = 141°C.

| Analyse : $C_{21}H_{20}N_2O_3S_2$ | | | | | | | | |
|---|---|---|---|---|---|---|---|---|
| Calculé<br>Trouvé | C% | 61,14<br>61,0 | H% | 4,89<br>4,8 | N% | 6,79<br>6,7 | S% | 15,54<br>15,2 |

En opérant selon les modes opératoires indiqués précédemment à partir des produits appropriés, on a préparé les produits des exemples suivants :

**EXEMPLE 18 : 2 (alpha)-(méthoxyméthylène) 2'-éthyl (2,4'-bithiazol) 4-acétate de méthyle.**

**EXEMPLE 19 : (Z,E) 3-méthoxy 2-[méthyl [2-fluoro 1-oxo 3-[2-(trifluorométhyl) 4-thiazolyl] 2-propényl] amino] 2-propènoate de méthyle.**

**EXEMPLE 20 : alpha-(méthoxyméthylène) 2-[(2-phényl 4-thiazolyl) méthoxy] benzène acétate de méthyle.**

**EXEMPLE 21 : alpha (E)-(méthoxyméthylène) 2-(E)-[[2-(2-pentyl) 4-thiazolyl] éthényl] benzène acétate de méthyle.**

**EXEMPLE 22 : alpha (Z)-(méthoxyméthylène) 2-(E)-[[2-(2-pentyl) 4-thiazolyl] éthényl] benzène acétate de méthyle.**

**EXEMPLE 23 : (E) alpha-(méthoxyméthylène) 2-[(2-pentyl) 4-thiazolyl] méthoxy] benzène acétate de méthyle.**

EXEMPLE 24 : (Z) alpha-(méthoxyméthylène) 2-[(2-pentyl) 4-thiazolyl] méthoxy] benzène acétate de méthyle.

EXEMPLE 25 : alpha (E) (méthoxyméthylène) 2-[(2-(2-méthylpropen-1-yl) 4-thiazolyl] méthoxy] benzène acétate de méthyle.

EXEMPLE 26 : (Z) alpha-(méthoxyméthylène) 2-[(4-thiazolyl 2-méthyléthyl) méthoxy] benzène acétate de méthyle.

EXEMPLE 27 : (E) alpha-(méthoxyméthylène) 2-[(4-thiazolyl 2-méthyléthyl) méthoxy] benzène acétate de méthyle.

EXEMPLE 28 : (E) 2-[[2'-éthyl (2,4'-bithiazol)-4-yl] méthoxy] alpha-(méthoxyméthylèn) benzène acétate de méthyle.

EXEMPLE 29 : alpha (E)-(méthoxyméthylène) 2-(E)-[[2-(2-pentyl) 4-thiazolyl] éthényl] benzène acétate de méthyle.

EXEMPLE 30 : (E)-alpha-(méthoxyméthylène) 2-[(4-thiazolyl 2-méthyléthyl] éthoxy] benzène acétate de méthyle.

EXEMPLE 31 : E,E alpha-(méthoxyméthylène) 2-[2-(2-méthyléthyl 4-thiazolyl) éthényl] benzène acétate de méthyle.

EXEMPLE 32 : E,Z alpha-(méthoxyméthylène) 2-[2-(2-méthyléthyl 4-thiazolyl) éthényl] benzène acétate de méthyle.

EXEMPLE 33 : alpha (Z)-(méthoxyméthylène) 2-(E)-[[2-(2-pentyl) 4-thiazolyl] éthényl] benzène acétate de méthyle.

EXEMPLE 34 : alpha (E)-(méthoxyméthylène) 2-(E)-[(2-méthyl 4-thiazolyl) éthényl] benzène acétate de méthyle.

EXEMPLE 35 : alpha (Z)-(méthoxyméthylène) 2-(E)-[(2-méthyl 4-thiazolyl) éthényl] benzène acétate de méthyle.

EXEMPLE 36 : alpha (E)-(méthoxyméthylène) 2-(E)-[[2-(2-méthyl 1-propényl) 4-thiazolyl] éthényl] benzène acétate de méthyle.

EXEMPLE 37 : alpha (Z)-(méthoxyméthylène) 2-(E)-[[2-(2-méthyl) 1-propényl) 4-thiazolyl] éthényl] benzène acétate de méthyle.

EXEMPLE 38 : alpha-(méthoxyméthylène) 2-[(2-méthyl 4-thiazolyl] méthoxy] benzène acétate de méthyle.

EXEMPLE 39 : alpha-(méthoxyméthylène) 2-[(2-chloro 4-thiazolyl) méthoxy] benzène acétate de méthyle.

EXEMPLE 40 : alpha-(méthoxyméthylène) 2-[(2-méthoxy 4-thiazolyl) méthoxy] benzène acétate de méthyle.

EXEMPLE 41 : alpha-(méthoxyméthylène) 2-[(2-méthylthio 4-thiazolyl) méthoxy] benzène acétate de méthyle.

EXEMPLE 42 : alpha (E)-(méthoxyméthylène) 2-(E)-[[2-(2-méthylthio 4-thiazolyl) éthényl] benzène acétate de méthyle.

**EXEMPLE 43 : alpha (E)-(méthoxyméthylène) 2-(E)-[[2-(2-chloro4-thiazolyl) éthényl] benzène acétate de méthyle.**

**EXEMPLE 44 : (E) alpha-(méthoxyméthylène) 2-[[4-oxazolylmé-thyl] méthoxy] benzène acétate de méthyle.**

**EXEMPLE 45 : E,E alpha-(méthoxyméthylène) 2-[2-(2-méthyl 4-oxazolyl) éthényl] benzène acétate de méthyle.**

**EXEMPLE 46 : alpha (E)-(méthoxyméthylène) 2-(E)-[[2-(2-méthoxy 4-thiazolyl) éthényl] benzène acétate de méthyle.**

Les produits utilisés au depart des exemples 19 à 46 ont été préparés comme indiqué dans les préparations décrites ci-après selon un mode opératoire analogue.

**PREPARATION 11 : 2-[(2-phényl 4-tiazolyl) méthoxy] benzène acétate de méthyle.**

**STADE A :** (2-phényl 4-thiazolyl) carboxylate d'éthyle.

On opère comme au stade B de la préparation 1 à partir de 100 g de thiobenzamide. Après chromatographie sur silice (éluant : n-hexane-acétate d'éthyle 8-2), on obtient 150 g du produit recherché.

| SPECTRE IR ($CHCl_3$) : | |
|---|---|
| C=O<br>aromatique | 1724 $cm^{-1}$<br>1438 $cm^{-1}$ |

**STADE B :** (2-phényl 4-thiazolyl) méthanol.

On opère comme au stade C de la préparation 1 à partir de 150 g du produit obtenu au stade A ci-dessus, en utilisant 64 g d'hydrure de bore et de sodium. On obtient 122 g du produit attendu. F = 69,7 °C.

| SPECTRE IR ($CHCl_3$) : | |
|---|---|
| -OH<br>aromatique<br>hétérocycle | 3610 $cm^{-1}$<br>1524 $cm^{-1}$<br>1503 $cm^{-1}$ |

**STADE C :** 2-[(2-phényl 4-tiazolyl) méthoxy] benzène acétate de méthyle.

a) A un mélange de 4,78 g du produit obtenu au stade B ci-dessus, 50 $cm^3$ de tétrahydrofuranne et 1,9 $cm^3$ de chlorure de méthanesulfonyle, on ajoute à 0 °C une solution de 3,5 $cm^3$ de triéthylamine dans 35 $cm^3$ de tétrahydrofuranne. On agite 1 heure à 0 °C, filtre et concentre à sec et obtient 6,8 g de mésylate intermédiaire.

b) A un solution de 2-hydroxyphénylacétate de méthyle dans 40 $cm^3$ de tétrahydrofuranne, on ajoute lentement à 0 °C 1,2 g d'hydrure de sodium à 50% dans l'huile. On laisse la température remonter à 20 °C et ajoute une solution de 6,8 g de mésylate obtenue précédemment dans 60 $cm^3$ de tétrahydrofuranne. On agite 2 heures 30 minutes à 20/25 °C, verse le mélange réactionnel sur une solution d'acide chlorhydrique N et de glace, extrait avec du chlorure de méthylène, sèche, filtre et évapore à sec sous pression réduite. On chromatographie le résidu obtenu (13 g) sur silice (éluant : n-hexane-acétate d'éthyle 7-3) et obtient 3,5 g du produit recherché. F = 65,5 °C.

SPECTRE IR ($CHCl_3$) :

| | |
|---|---|
| =O | : 1733 $cm^{-1}$ |
| aromatique + système conjugué | 1606, 1593, 1526 et 1498 $cm^{-1}$ |

**PREPARATION 12 : 2-[[2'-éthyl (2,4'-bithiazol) 4-yl] méthoxy] benzène acétate de méthyle.**

On opère comme au stade C de la préparation 11 à partir de 4,2 g du produit obtenu au stade F de la préparation 4 en utilisant 1,44 $cm^3$ de chlorure de méthanesulfonyle et 3,4 g de 2-hydroxyphényl acétate de méthyle. Après chromatographie sur silice (éluant : chlorure de méthylène-acétone 99-1), on obtient 3,1 g du produit recherché.

SPECTRE IR ($CHCl_3$) :

| | |
|---|---|
| >=O | : 1734 $cm^{-1}$ |
| aromatique + système conjugué | 1604, 1588 et 1491 $cm^{-1}$ |

**PREPARATION 13 : 2-[2-[2-(2-pentyl) 4-thiazolyl] éthényl] benzène acétate de méthyle.**

**STADE A :** n-pentyl thioamide.

On opère comme au stade A de la préparation 1 à partir de 23 g d'amylacétamide en utilisant 10,7 g de pentasulfure de phosphore. Après chromatographie sur silice (éluant : chlorure de méthylène-acétate d'éthyle 9-1), on obtient 18,3 g du produit recherché. F = 52,9 °C.

| SPECTRE IR ($CHCl_3$) : | |
|---|---|
| =C-$NH_2$ | 3486, 3380 $cm^{-1}$ |
| $NH_2$ déf. | 1604 $cm^{-1}$ |

**STADE B :** 2-n-pentyl 4-thiazolyl carboxylate d'éthyle.

On opère comme au stade B de la préparation 1 à partir de 36 g de produit obtenu comme au stade précédent en utilisant 60 g de bromopyruvate d'éthyle. Après chromatographie sur silice (éluant : hexane-acétate d'éthyle 7-3), on obtient 49 g du produit recherché.

| SPECTRE IR ($CHCl_3$) : | |
|---|---|
| > =O | 1722 $cm^{-1}$ |
| hétérocycle | 1608, 1504 $cm^{-1}$ |

**STADE C :** 2-n-pentyl 4-thiazolyl méthanol.

On opère comme au stade C de la préparation 1 à partir de 49 g du produit obtenu au stade B ci-dessus en utilisant 20 g d'hydrure de bore et de sodium. On obtient 40 g de produit attendu.

| SPECTRE IR ($CHCl_3$) : | |
|---|---|
| -OH<br>hétéroatome | 3608 $cm^{-1}$<br>1530 $cm^{-1}$ |

**STADE D :** 2-pentyl 4-formyl thiazole.

On opère comme au stade D de l'exemple 1 à partir de 30 g du produit obtenu au stade C ci-dessus en utilisant 130 g de bioxyde de manganèse. On obtient 25 g du produit recherché.

| SPECTRE IR ($CHCl_3$) : | |
|---|---|
| >-OH<br>hétérocycle | 1698 $cm^{-1}$<br>1488 $cm^{-1}$ |

**STADE E :** 2-[2-(E) [2-[2-pentyl] 4-thiazolyl] éthényl] benzène carbonitrile.

On opère comme à l'exemple 16 à partir de 9,2 g du produit obtenu au stade D ci-dessus en utilisant 13 g de 2-diéthylphosphonate benzène carbonitrile (obtenu selon EP 0 203 606). Après chromatographie sur silice (éluant : hexane-acétate d'éthyle 7-3), on obtient 8,43 g du produit recherché.

SPECTRE IR ($CHCl_3$) :
C=N : 2225 $cm^{-1}$
C=C : 1634 $cm^{-1}$
aromatique + hétérocycle : 1596, 1566, 1504 $cm^{-1}$
C=CH : 964 $cm^{-1}$

**STADE F :** 2-[2-(E) [2-[2-pentyl] 4-thiazolyl] éthényl] benzaldéhyde.

A un mélange de 8 g du produit obtenu au stade E ci-dessus et 100 $cm^3$ de toluène, on ajoute à -50 °C 40 $cm^3$ d'une solution 1,2M d'hydrure de diisobutylaluminium dans le toluène. On agite 2 heures à -50/-55 °C, verse le mélange réactionnel dans une solution de 300 $cm^3$ d'eau et 100 $cm^3$ de tartrate double de potassium et sodium. On extrait à l'éther, sèche, amène à sec sous pression réduite et obtient 9 g de produit que l'on chromatographie sur silice (éluant : hexane-acétate d'éthyle 8-2). On obtient 73 g du produit recherché.

SPECTRE IR ($CHCl_3$) :
>=O : 1698 $cm^{-1}$
C=C + aromatique + hétérocycle : 1630, 1596, 1566, 1504 $cm^{-1}$

**STADE G :** 2-pentyl 4-[2-[2-[2-(méthylthio) 2-(méthylsulfinyl) éthényl] phényl] éthényl] thiazole.

A une solution de 7 g du produit obtenu au stade F ci-dessus, 50 $cm^3$ de tétrahydrofuranne et 3,2 $cm^3$ de sulfure de méthyl méthyl sulfinyl méthyle, on ajoute 4,9 $cm^3$ d'hydroxyde de benzyltriméthylammonium

en solution à 35% dans le méthanol. On chauffe 3 heures au reflux, verse dans l'eau, extrait avec du chlorure de méthylène, sèche et évapore à sec sous pression réduite. On chromatographie les 10 g de résidu sur silice (éluant : hexane-acétate d'éthyle 6-4) et obtient 6,85 g du produit recherché.

<u>SPECTRE IR ($CHCl_3$)</u> :

| | |
|---|---|
| C=C | : 1634 $cm^{-1}$ |
| aromatique + hétérocycle | 1599, 1558, 1504 $cm^{-1}$ |
| S --> O | : 1055 $cm^{-1}$ |

**<u>STADE H</u> :** 2-[2-[2-(2-pentyl) 4-thiazolyl] éthényl] benzène acétate de méthyle.

A une solution de 6,8 g du produit obtenu au stade G ci-dessus dans 40 $cm^3$ de méthanol à 0°/+5°C, on ajoute lentement une solution de méthanol chlorhydrique préparée par ajout de 27 $cm^3$ de chlorure d'acétyle dans 110 $cm^3$ de méthanol à 0°C. On agite 4 heures à O°/+5°C, verse dans l'eau puis neutralise par addition de bicarbonate de sodium. On extrait avec du chlorure de méthylène, sèche et amène à sec sous pression réduite. On obtient 5,1 g du produit recherché.

<u>SPECTRE IR ($CHCl_3$)</u> :

| | |
|---|---|
| >=O | : 1733 $cm^{-1}$ |
| C=C + aromatique + hétérocycle | 1634 (f), 1600, 1574, 1504 $cm^{-1}$ (f) |

**<u>PREPARATION 14</u> : <u>2-[2 (E) [2-(2-méthyléthyl) 4-thiazolyl] éthényl] benzène acétate de méthyle.</u>**

**<u>STADE A</u> :** thioisobutyramide.

On opère comme au stade A de la préparation 1 à partir de 95,8 g d'isobutyramide en utilisant 58,2 g de pentasulfure de phosphore. Après chromatographie sur silice (éluant : hexane-acétate de méthyle 7-3), on obtient 61,6 g du produit recherché.

| <u>SPECTRE IR ($CHCl_3$)</u> : | |
|---|---|
| =C-$NH_2$<br>$NH_2$ | 3490, 3377 $cm^{-1}$<br>1604 $cm^{-1}$ |

**<u>STADE B</u> :** (2-méthyléthyl 4-thiazolyl) carboxylate d'éthyle.

On opère comme au stade B de la préparation 1 à partir de 61 g du produit obtenu au stade A ci-dessus en utilisant 74 $cm^3$ de bromopyruvate d'éthyle. Après chromatographie sur silice (éluant : hexane-acétate d'éthyle 7-3), on obtient 87,6 g du produit recherché.

| <u>SPECTRE IR ($CHCl_3$)</u> : | |
|---|---|
| >C=O<br>hétérocycle | 1723 $cm^{-1}$<br>1604, 1504 $cm^{-1}$ |

**STADE C :** (2-méthyléthyl 4-thiazolyl) méthanol.

On opère comme au stade C de la préparation 1 à partir de 87,6 g du produit obtenu au stade B ci-dessus et en utilisant 44 g d'hydrure de bore et de sodium. On obtient 61,4 g du produit attendu.

| SPECTRE IR ($CHCl_3$) : | |
|---|---|
| -OH<br>système conjugué | 3612 $cm^{-1}$<br>1530 $cm^{-1}$ |

**STADE D :** (2-méthyléthyl 4-thiazolyl) méthanal.

On opère comme au stade D de la préparation 1 à partir de 47 g du produit obtenu au stade C ci-dessus et en utilisant 130 g de bioxyde de manganèse. Après chromatographie sur silice (éluant : hexane-acétate d'éthyle 4-6), on obtient 33,5 g du produit recherché.

SPECTRE IR ($CHCl_3$) :

absence d'OH

>C=O : 1698 $cm^{-1}$ du type aldéhyde

**STADE E :** 2-[2-(E) [2-[2-méthyléthyl] 4-thiazolyl] éthényl] benzène carbonitrile.

On opère comme au stade E de la préparation 13 à partir de 16,7 g du produit obtenu au stade précédent en utilisant 28 g de 2-diéthylphosphonate benzène carbonitrile (obtenu selon EP 0 203 606). Après chromatographie sur silice (éluant : chlorure de méthylène-hexane 9-1), on obtient 20,2 g du produit recherché.

SPECTRE IR ($CHCl_3$) :

C=N : 2225 $cm^{-1}$

C=C : 1636 $cm^{-1}$

aromatique + hétérocycle : 1596, 1566, 1504 $cm^{-1}$

**STADE F :** 2-[2-(E) [2-[2-méthyléthyl] 4-thiazolyl] éthényl] benzaldéhyde.

On opère comme au stade F de la préparation 13 à partir de 20 g du produit obtenu au stade E ci-dessus en utilisant 98 $cm^3$ d'hydrure de diisobutylaluminium. Après chromatographie sur silice (éluant : chlorure de méthylène-hexane 9-1), on obtient 17,7 g du produit recherché.

SPECTRE IR ($CHCl_3$) :

C=O : 1688 $cm^{-1}$

C=C : 1631 $cm^{-1}$

aromatique + hétérocycle : 1596, 1568, 1503 $cm^{-1}$

**STADE G** : 2-méthyléthyl 4-[2 (E) [2-[2 (E ou Z) (méthylthio) 2-(méthylsulfinyl) éthényl] phényl] éthényl] thiazole.

On opère comme au stade G de la préparation 13 à partir de 12,86 g du produit obtenu au stade F ci-dessus et en utilisant 6,5 $cm^3$ de méthyl méthyl sulfinyl méthyle sulfure. Après chromatographie sur silice (éluant : acétate d'éthyle-hexane 6-4), on obtient 13 g du produit recherché.

**SPECTRE IR ($CHCl_3$) :**

S --> O ~ 1055 $cm^{-1}$

C=C + aromatique : 1632, 1595, 1502 $cm^{-1}$

C=O : 1730 $cm^{-1}$

**STADE H** : 2-[2 (E) [2-(2-méthyléthyl) 4-thiazolyl] éthényl] benzène acétate de méthyle.

On opère comme au stade H de la préparation 13 à partir de 12,92 g du produit obtenu au stade G ci-dessus. Après chromatographie sur silice (éluant : hexane-acétate d'éthyle 7-3), on obtient 8 g du produit recherché.

**SPECTRE IR ($CHCl_3$) :**

$C(=O)-OCH_3$ : 1738, 1439 $cm^{-1}$

C=C + aromatique : 1634, 1599, 1505 $cm^{-1}$

**PREPARATION 15 : 2-[(2-méthyl 4-thiazolyl) méthoxy] benzène acétate de méthyle.**

**STADE A** : (2-méthyl 4-thiazolyl) carboxylate de méthyle.

On opère comme au stade B de la préparation 1 à partir de 100 g de thioacétamide en utilisant 167,5 $cm^3$ de bromopyruvate d'éthyle, on obtient 140 g du produit recherché.

**STADE B** : (2-méthyl 4-thiazolyl) méthanol.

On opère comme au stade C de la préparation 1 à partir de 70 g du produit obtenu au stade A ci-dessus en utilisant 38,7 g d'hydrure de bore et de sodium et obtient 31 g du produit recherché.

**STADE C** : 2-[(2-méthyl 4-thiazolyl) méthoxy] benzène acétate de méthyle.

On opère comme au stade C de la préparation 11 à partir de 6 g du produit obtenu au stade B ci-dessus et en utilisant 3,6 g de chlorure de méthanesulfonyle. Après chromatographie sur silice (éluant : hexane-acétate d'éthyle 7-3), on obtient 3 g de produit recherché. F = 80 °C.

**SPECTRE IR ($CHCl_3$) :**

=O : 1734 $cm^{-1}$

aromatique + thiazole : 1606, 1592, 1540, 1496 $cm^{-1}$

Les tableaux ci-dessous résument les principales caractéristiques des produits obtenus dans les exemples (spectres RMN permettant de caractériser les différents isomères ; les points de fusion lorsque les produits consernés ont un point de fusion.

**TABLEAU I**

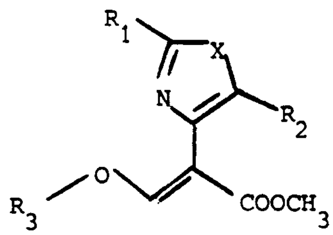

| EX | X | R1 | R2 | R3 | isomère | F°C | SD |
|---|---|---|---|---|---|---|---|
| 8 | S | 4ClC6H4 | H | CH2-O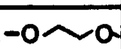 | Z | - | 8,13 |
| 4 | S | S-CH3 | H | CH2-O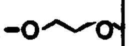 | Z | - | 7,98 |
| 7 | S | 4-ClC6H4 | H | CH3 | Z | 109 | 7,9 |
| 3 | S | S-CH3 | H | CH3 | Z | 50 | 7,84 |
| 3 | S | S-CH3 | H | CH3 | E | - | 7,59 |
| 7 | S | 4-ClC6H4 | H | CH3 | E | - | 7,64 |
| 8 | S | 4-ClC6H4 | H | CH2-O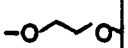 | E | 50 | 7,85 |
| 4 | S | S-CH3 | H | CH2-O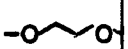 | E | - | 7,79 |
| 18 | S | i | H | CH3 | E | 72 | - |

i = 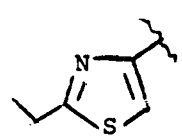

**TABLEAU II**

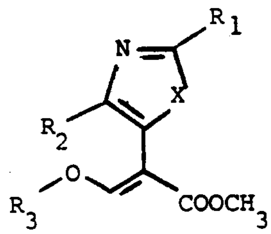

| EX | X | R1 | R2 | R3 | isomère | F°C | SD |
|---|---|---|---|---|---|---|---|
| 9 | S | 4-CyprC6H4 | 4-ClC6H4 | CH3 | E | 153 | 6,73 |
| 10 | S | 4-CyprC6H4 | 4-ClC6H4 | 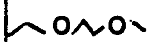 | E | 82 | 6,97 |
| 9 | S | 4-CyprC6H4 | 4-ClC6H4 | CH3 | Z | 114 | 7,53 |
| 10 | S | 4-CyprC6H4 | 4-ClC6H4 | 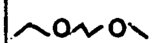 | Z | - | 7,77 |
| 11 | S | SCH3 | CH3 | CH3 | Z | - | 7,6 |
| 11 | S | SCH3 | CH3 | CH3 | E | - | 6,72 |
| 12 | S | SCH3 | CH3 | 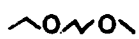 | Z | - | 7,84 |
| 12 | S | SCH3 | CH3 | 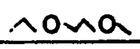 | E | - | 6,96 |

## TABLEAU III

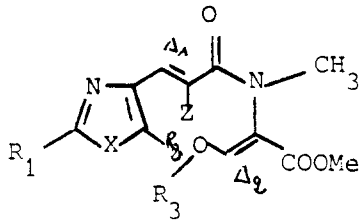

| EX | X | R1 | R2 | Z | R3 | Δ 1 | Δ 2 | F°C | δ Δ 2 |
|---|---|---|---|---|---|---|---|---|---|
| 1 | S | CF3 | H | H | CH3 | E | Z | 104 | 7,49 - 7,52 |
| 14 | S | i | H | F | CH3 | Z | Z | 99 | 7,35 |
| 5 | S | H | H | H | CH3 | E | Z | 108 | 7,46 |
| 6 | S | H | H | H | 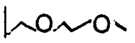 | E | Z | 86 | 7,72 |
| 13 | S | i | H | H | CH3 | E | Z | - | 7,36 |
| 15 | S | H | H | F | CH3 | Z | Z | - | 7,34 |
| 2 | S | CF3 | H | H | 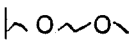 | E | Z | 76 | 7,77 |
| 19 | S | CF3 | H | F | CH3 | Z | E | | |

i = 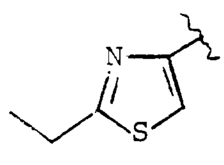

**TABLEAU IV**

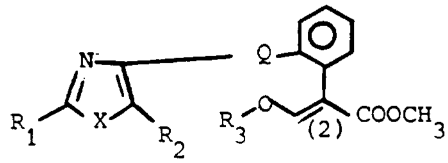

| EX | X | R1 | R2 | Q | R3 | DQ | D2 | F°C | SD |
|---|---|---|---|---|---|---|---|---|---|
| 17 | S | i | H | -CH=CH- | CH3 | E | E | 141 | 7,65 |
| 16 | S | H | H | -CH=CH- | CH3 | E | E | 106 | 7,63 |
| 20 | S | C6H5 | H | CH2-O | CH3 | - | E | 89 | 7,54 |
| 21 | S | C6H5 | H | -CH=CH- | CH3 | E | E | 115 | 7,66 |
| 22 | S | C6H5 | H | -CH=CH- | CH3 | E | Z | 131 | 6,58 |
| 23 | S | nC5H11 | H | CH2-O | CH3 | - | E | - | 7,52 |
| 24 | S | nC5H11 | H | CH2-O | CH3 | - | Z | - | 6,59 |
| 25 | S | CH=<$^{CH_3}_{CH_3}$ | H | CH2-O | CH3 | - | E | - | 7,53 |
| 26 | S | iC3H7 | H | CH2-O | CH3 | - | Z | - | 6,59 |
| 27 | S | H | H | CH2-O | CH3 | - | E | 67 | 7,53 |
| 28 | S | i | H | CH2-O | CH3 | - | E | 112 | 7,54 |
| 29 | S | nC5H11 | H | -CH=CH- | CH3 | E | E | - | 7,63 |
| 30 | S | iC3H7 | H | CH2-O | CH3 | - | E | - | 7,52 |
| 31 | S | iC3H7 | H | -CH=CH- | CH3 | E | E | 92 | 7,63 |
| 32 | S | iC3H7 | H | -CH=CH- | CH3 | E | Z | - | 6,56 |
| 33 | S | nC5H11 | H | -CH=CH- | CH3 | E | Z | - | 6,55 |
| 34 | S | CH3 | H | -CH=CH- | CH3 | E | E | 115 | 7,63 |
| 35 | S | CH3 | H | -CH=CH- | CH3 | E | Z | 136 | 6,56 |
| 36 | S | -CH=<$^{CH_3}_{CH_3}$ | H | -CH=CH- | CH3 | E | E | 103 | 7,62 |
| 37 | S | -CH=<$^{CH_3}_{CH_3}$ | H | -CH=CH- | CH3 | E | Z | 134 | 6,55 |
| 38 | S | CH3 | H | CH2O | CH3 | - | E | - | 7,52 |
| 39 | S | Cl | H | CH2O | CH3 | - | E | 97 | 7,51 |
| 40 | S | OCH3 | H | CH2O | CH3 | - | E | 83 | 7,51 |
| 41 | S | SCH3 | H | CH2O | CH3 | - | E | 89 | 7,53 |
| 42 | S | SCH3 | H | -CH=CH- | CH3 | E | E | 124 | 7,63 |
| 43 | S | Cl | H | -CH=CH- | CH3 | E | E | 114 | 7,64 |
| 44 | O | CH3 | H | CH2O | CH3 | - | E | - | 7,49 |
| 45 | O | CH3 | H | -CH=CH- | CH3 | E | E | 88 | 7,62 |
| 46 | S | OCH3 | H | -CH=CH- | CH3 | E | E | 112 | 7,62 |
| 47 | S | 3.4Cl2C6H3 | H | CH2O | CH3 | - | E | 130 | 7,55 |
| 48 | S | 3.4Cl2C6H3 | H | CH2O | CH3 | - | Z | 139 | 6,61 |
| 49 | S | 3.4Cl2C6H3 | H | -CH=CH- | CH3 | E | E | 183 | 7,67 |
| 50 | S | 3.4Cl2C6H3 | H | -CH=CH- | CH3 | Z | Z | 137 | 6,81 |

i = 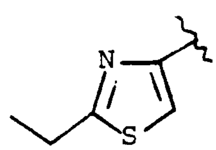

**TABLEAU IV**
**(suite)**

| X | R1 | R2 | Q | R3 | DQ | D2 |
|---|---|---|---|---|---|---|
| S | 2Cl-C6H4 | H | CH2O | CH3 | - | E |
| S | " | H | CH=CH | CH3 | E | E |
| S | 3Cl-C6H4 | H | CH2O | CH3 | - | E |
| S | " | H | CH=CH | CH3 | E | E |
| S | 4Cl-C6H4 | H | CH2O | CH3 | - | E |
| S | " | H | CH=CH | CH3 | E | E |
| S | 2.6Cl2C6H3 | H | CH2O | CH3 | - | E |
| S | " | H | CH=CH | CH3 | E | E |
| S | 2.4Cl2C6H3 | H | CH2O | CH3 | - | E |
| S | " | H | CH=CH | CH3 | E | E |
| S | 3.5Cl2C6H3 | H | CH2O | CH3 | - | E |
| S | " | H | CH=CH | CH3 | E | E |
| S | 3OCH3,4ClC6H3 | H | CH2O | CH3 | - | E |
| S | " | H | CH=CH | CH3 | E | E |
| S | 3CF3,4PrC6H3 | H | CH2O | CH3 | - | E |
| S | " | H | CH=CH | CH3 | E | E |
| S | 2CF3-C6H4 | H | CH2O | CH3 | - | E |
| S | " | H | CH=CH | CH3 | E | E |
| S | 3CF3-C6H4 | H | CH2O | CH3 | - | E |
| S | " | H | CH=CH | CH3 | E | E |
| S | 4CF3-C6H4 | H | CH2O | CH3 | - | E |
| S | " | H | CH=CH | CH3 | E | E |
| S | 2OCH3-C6H4 | H | CH2O | CH3 | - | E |
| S | " | H | CH=CH | CH3 | E | E |
| S | 3OCH3-C6H4 | H | CH2O | CH3 | - | E |
| S | " | H | CH=CH | CH3 | E | E |
| S | 4OCH3-C6H4 | H | CH2O | CH3 | - | E |
| S | " | H | CH=CH | CH3 | E | E |
| S | 2F-C6H4 | H | CH2O | CH3 | - | E |
| S | " | H | CH=CH | CH3 | E | E |
| S | 3F-C6H4 | H | CH2O | CH3 | - | E |
| S | " | H | CH=CH | CH3 | E | E |
| S | 4F-C6H4 | H | CH2O | CH3 | - | E |
| S | " | H | CH=CH | CH3 | E | E |
| S | 4F-3BrC6H3 | H | CH2O | CH3 | - | E |
| S | " | H | CH=CH | CH3 | E | E |
| S | 4F-6BrC6H3 | H | CH2O | CH3 | - | E |
| S | " | H | CH=CH | CH3 | E | E |
| S | 2,4-F2C6H3 | H | CH2O | CH3 | - | E |
| S | " | H | CH=CH | CH3 | E | E |
| S | 2-pyridyl | H | CH=CH | CH3 | E | E |
|  | " | H | CH2O | CH3 | - | E |
| S | 3-pyridyl | H | CH=CH | CH3 | E | E |
| S | " | H | CH2O | CH3 | - | E |
| S | 4-pyridyl | H | CH=CH | CH3 | E | E |
| S | " | H | CH2O | CH3 | - | E |
| S | 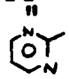 | H | CH=CH | CH3 | E | E |
| S | " | H | CH2O | CH3 | - | E |

**TABLEAU IV**

**(suite)**

| X | R1 | R2 | Q | R3 | DQ | D2 |
|---|---|---|---|---|---|---|
| S | 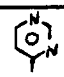 | H | CH=CH | CH3 | E | E |
| S | " | H | CH2O | CH3 | - | E |
| S | 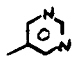 | H | CH=CH | CH3 | E | E |
| S | " | H | CH2O | CH3 | - | E |
| S | 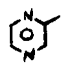 | H | CH=CH | CH3 | E | E |
| S | " | H | CH2O | CH3 | - | E |
| S | 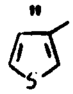 | H | CH=CH | CH3 | E | E |
| S | " | H | CH2O | CH3 | - | E |
| S | 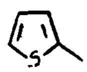 | H | CH=CH | CH3 | E | E |
| S | " | H | CH2O | CH3 | - | E |
| S | 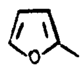 | H | CH=CH | CH3 | E | E |
| S | " | H | CH2O | CH3 | - | E |
| S | 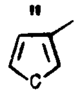 | H | CH=CH | CH3 | E | E |
| S | " | H | CH2O | CH3 | - | E |
| S | 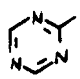 | H | CH=CH | CH3 | E | E |
| S | " | H | CH2O | CH3 | - | E |
| S | 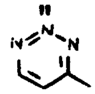 | H | CH=CH | CH3 | E | E |
| S | " | H | CH2O | CH3 | - | E |
| S | 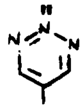 | H | CH=CH | CH3 | E | E |
| S | " | H | CH2O | CH3 | - | E |
| S | 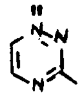 | H | CH=CH | CH3 | E | E |
| S | " | H | CH2O | CH3 | - | E |
| S | 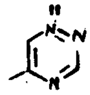 | H | CH=CH | CH3 | E | E |
| S | " | H | CH2O | CH3 | - | E |

TABLEAU IV

(suite)

| X | R1 | R2 | Q | R3 | DQ | D2 |
|---|---|---|---|---|---|---|
| S | 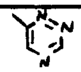 | H | CH=CH | CH3 | E | E |
| S | " | H | CH2O | CH3 | - | E |
| S | 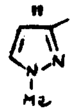 | H | CH=CH | CH3 | E | E |
| S | " | H | CH2O | CH3 | - | E |
| S | 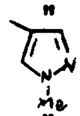 | H | CH=CH | CH3 | E | E |
| S | " | H | CH2O | CH3 | - | E |
| S | 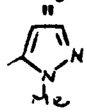 | H | CH=CH | CH3 | E | E |
| S | " | H | CH2O | CH3 | - | E |
| S | 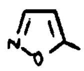 | H | CH=CH | CH3 | E | E |
| S | " | H | CH2O | CH3 | - | E |
| S | 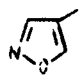 | H | CH=CH | CH3 | E | E |
| S | " | H | CH2O | CH3 | - | E |
| S | 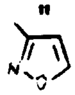 | H | CH=CH | CH3 | E | E |
| S | " | H | CH2O | CH3 | - | E |
| S | 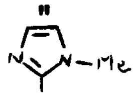 | H | CH=CH | CH3 | E | E |
| S | " | H | CH2O | CH3 | - | E |
| S | 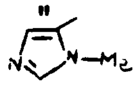 | H | CH=CH | CH3 | E | E |
| S | " | H | CH2O | CH3 | - | E |
| S | 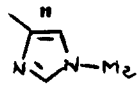 | H | CH=CH | CH3 | E | E |
| S | " | H | CH2O | CH3 | - | E |
| S | 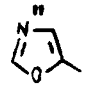 | H | CH=CH | CH3 | E | E |
| S | " | H | CH2O | CH3 | - | E |
| S | 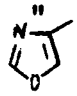 | H | CH=CH | CH3 | E | E |
| S | " | H | CH2O | CH3 | - | E |
| S | 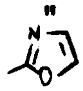 | H | CH=CH | CH3 | E | E |
| S | " | H | CH2O | CH3 | - | E |

## ACTIVITE BIOLOGIOUE FONGICIDE

### a) Essais Botrytis cinerea sur vigne.

Les jeunes plants de vigne issus de boutures (variété Grenache N, clone 70) sont cultivés en serre (température de jour : 30°C, température de nuit : 25°C) sur mélange terre/terreau/sable, (1/3-1/3-1/3). Deux jours avant l'essai, les plants sont transportés en chambre de culture (mêmes conditions de température, humidité : 60% le jour, 80% la nuit). Le produit est dissous dans la "matrice A" à une concentration de 500 ppm juste avant l'utilisation. Le traitement se fait par pulvérisation de la solution sur les feuilles jusqu'à rétention maximale. Les spores de Botrytis cinerea sont mises en suspension dans du jus de carottes dilué, à raison de 50.000 spores par ml. La contamination s'effectue par dépôt de la suspension de spores sous forme de gouttes (20 microlitres) à la surface abaxiale des feuilles. Dans l'essai préventif, le traitement est effectué un jour avant la contamination. Les plants sont ensuite maintenus dans la chambre de culture, aux mêmes conditions que précédemment. La lecture est faite neuf jours après la contamination, par mesure des surfaces nécrosées. L'efficacité du produit est calculée par rapport à un témoin non traité.

### b) Essais Plasmopara viticola.

Le matériel végétal utilisé est le même que celui utilisé dans l'essai A, et cultivé dans les mêmes conditions. Le traitement s'effectue de la même façon également. La contamination est faite avec une suspension de zoosporanges de Plasmopara viticola prélevés immédiatement avant l'essai (50.000 zoosporanges par ml). Les gouttes de suspension (20 microlitres) sont déposées à la face abaxiale des feuilles. Les plants sont ensuite maintenus pendant 24 heures dans une atmosphère saturée en humidité, puis ramenés à l'humidité de la chambre de culture (60% le jour, 80% la nuit).

La lecture s'effectue dix jours après la contamination, par mesure du développement des îlots de conidiophores à la surface abaxiale des feuilles. L'efficacité du produit est calculée par rapport à un témoin non traité.

### c) Essais Erysiphe graminis hordei.

Les grains d'orge (variété Pression) sont mis à germer dans le mélange terre-terreau-sable (1/3-1/3-1/3) et cultivés en serre. Les produits sont dissous dans la "matrice A" juste avant l'essai, à une concentration de 500 ppm. Le traitement s'effectue par pulvérisation de la solution de produit sur les plants d'orge âgés de dix jours, jusqu'à rétention maximale. La contamination par les conidies d'Erysiphe graminis hordei est faite trois jours après le traitement. Les plants sont maintenus en salle climatisée (température de jour : 23°C, température de nuit : 18°C). Sept jours après la contamination, on mesure l'étendue du feutrage conidien sur la première et la deuxième feuille de chaque plant. L'efficacité du produit est calculée par rapport à un témoin non traité.

### d) Essais Gaeumannomyces graminis tritici.

Préparation de l'inoculum : 200 g de grains d'avoine autoclavés sont contaminés avec Gaeumannomyces graminis tritici, et conservés pendant cinq semaines à une température de 22°C, et à l'obscurité. L'inoculum est ensuite séché, réduit en poudre et conservé sous cette forme. Au jour de l'essai, la vermiculite lavée et humide est contaminée avec l'inoculum (1% en poids d'inoculum) et répartie dans les pots de culture (100 g par pot). Une couche de 2 cm de vermiculite non contaminée est déposée à la surface des pots.

Les grains de blé (variété Cappelle) sont semés dans cette couche supérieure. Les produits sont dissous dans la "matrice A" à la concentration de 50 ppm. Le traitement se fait par arrosage de chaque pot avec 30 ml de la solution de produit. Les pots sont maintenus en chambre de culture (température de jour : 21°C, température de nuit : 16°C). Le poids des parties aériennes et des parties racinaires de chaque plant est mesuré trois semaines après le semis. L'efficacité du traitement est calculée par rapport à un témoin non contaminé et à un témoin contaminé non traité.

**e) Essais Puccinia recondita tritici.**

Les grains de blé (variété Festival) sont mis à germer dans le mélange terre-terreau-sable (1/3,1/3,1/3). Les plants sont cultivés en serre. Les produits sont dissous dans la "matrice A" juste avant l'essai, à une concentration de 500 ppm. Le traitement s'effectue par pulvérisation de la solution de produit sur les plants de blé âgés de neuf jours, jusqu'à rétention maximale. La contamination par les urédospores de Puccinia recondita tritici est faite le lendemain du traitement. Les plants sont maintenus en salle climatisée (température de jour : 22 °C, température de nuit : 18 °C). Sept jours après la contamination, on mesure la densité des spores sur les deux premières feuilles de chaque plant. L'efficacité du produit est calculée par rapport à un témoin non traité.

Propriétés biologiques.

| Propriétés fongicides. | | | | | | | |
|---|---|---|---|---|---|---|---|
| Ex. | Botrytis (pre) | Palsmopara (pre) | Erysiphe | | Gacumano | | Puccinia |
| | | | 1ère feuille | 2ème feuille | feuille | racine | |
| 1 | | | 60 | | | | 80 |
| 5 | - | 10 | 30 | 50 | 10 | 100 | |
| 14 | - | 40 | 90 | 70 | 0 | 0 | 40 |
| 15 | 30 | 20 | 70 | 50 | 0 | 0 | 70 |
| 11(E) | 80 | 70 | - | - | 0 | 10 | 30 |
| 11(Z) | 20 | 100 | 50 | 30 | 0 | 0 | 30 |
| 17 | 70 | 70 | 60 | 40 | 0 | 0 | - |
| 16 | 80 | 100 | 70 | 50 | - | - | 50 |
| 20 | 40 | | 100 | 50 | - | - | 10 |
| 28 | 80 | | 100 | 40 | - | - | 30 |
| 29 | 100 | | 60 | 60 | - | - | 100 |
| 30 | 70 | - | - | 40 | - | - | 90 |
| 31 | 90 | - | - | - | - | - | 100 |
| 32 | 100 | - | 60 | 60 | - | - | 100 |
| 33 | 100 | - | 50 | 60 | - | - | 100 |
| 38 | 70 | - | 30 | 30 | - | - | 70 |

Exemples de compositions.

| Matrice A | | |
|---|---|---|
| | SOLVESSO 150 | 70,0 g |
| | NAPSOL PM1 | 850,0 g |
| | SURFAROX HRH 40C | 52,0 g |
| | ECD 1604 | 28,0 g |
| | | 1000,0 g |

| Matrice B | | |
|---|---|---|
| | SOLVESSO 150 | 64,5 g |
| | NAPSOL PM1 | 183,0 g |
| | PROPANEDIOL 1,2 | 158,5 g |
| | SURFAROX HRH 40C | 104,0 g |
| | ECD 1604 | 54,5 g |
| | EAU PERMUTEE | 435,5 g |
| | | 1000,0 g |

## Revendications

**Revendications pour les Etats contractants suivants : AT, BE, CH, DE, DK, FR, GB, GR, IT, LI, LU, NL, SE**

**1.** Les composés de formule générale (I) :

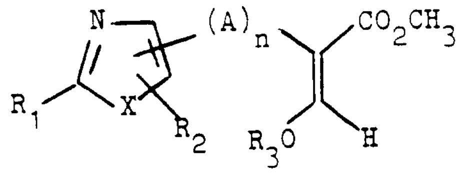

(I)

dans lesquels

- $R_1$ et $R_2$ identiques ou différents représentent un atome d'hydrogène, un atome d'halogène, un radical $CF_3$, un radical alkyle, alkényle, alkynyle ou thioalkyle renfermant jusqu'à 8 atomes de carbone, un radical alkoxy, thioalkoxy ou $SO_2$alkyle renfermant jusqu'à 8 atomes de carbone, un radical aryle, aryloxy ou thioaryle renfermant jusqu'à 18 atomes de carbone éventuellement substitué, un radical hétéroaryle ou hétéroaryloxy éventuellement substitué ou un radical hétérocyclique à 5 ou 6 chaînons éventuellement substitué,
- $R_3$ représente un radical alkyle, alkényle ou alkynyle renfermant jusqu'à 8 atomes de carbone ou un radical aryle renfermant jusqu'à 18 atomes de carbone ou un radical alkoxyalkoxyalkyle renfermant jusqu'à 14 atomes de carbone,
- X représente un atome d'oxygène ou de soufre ou un radical $NR_4$ dans lequel $R_4$ représente un atome d'hydrogène ou un radical alkyle, alkényle ou alkynyle, $SO_2$-alkyle, $SO_2$-alkényle ou $SO_2$-alkynyle renfermant jusqu'à 8 atomes de carbone ou un radical aryle ou $SO_2$-aryle renfermant jusqu'à 18 atomes de carbone éventuellement substitué,
- n représente le nombre O ou le nombre 1,
- A représente un radical choisi dans le groupe des radicaux suivants :

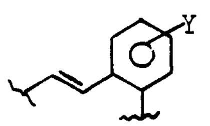 ; 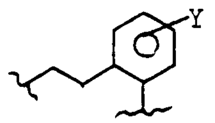 ; 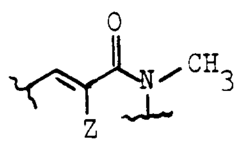 ; 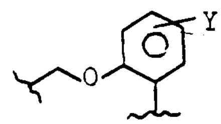

Y représentant un atome d'hydrogène, un atome d'halogène, un radical $CF_3$, un radical alkyle ou alkoxy renfermant juqu'à 8 atomes de carbone et Z représentant un atome d'hydrogène ou un atome d'halogène,

- la géométrie des doubles liaisons étant E ou Z ou un mélange de géométrie E et Z.

**2.** Les composés de formule (I) tels que définis à la revendication 1, dans lesquels le groupement

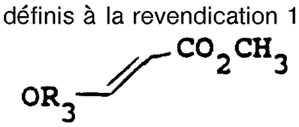

est tel que $CO_2CH_3$ et $OR_3$ sont en conformation trans l'un par rapport à l'autre.

**3.** Les composés de formule (I) tels que définis à la revendication 1 ou 2 dans lesquels X représente un atome de soufre.

**4.** Les composés de formule (I) tels que définis à l'une quelconque des revendications 1 à 3 dans lesquels A représente un radical

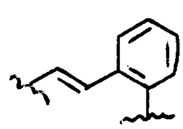

**5.** Les composés de formule (I) tels que définis à la revendication 4, dans lesquels la géométrie de la double liaison est E.

**6.** Les composés de formule (I) tels que définis à l'une quelconque des revendications 1 à 3, dans lesquels A représente un radical

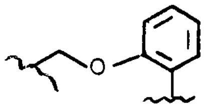

**7.** Les composés de formule (I) tels que définis à l'une quelconque des revendications 1 à 3, dans lesquels A représente un radical

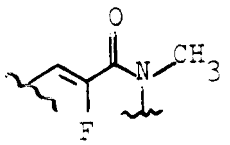

(A1)

ou

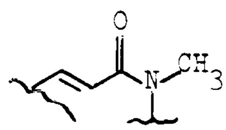

(A2)

**8.** Les composés de formule (I) tels que définis à la revendication 7, dans lesquels la géométrie de la double liaison est Z pour le radical $A_1$ et E pour le radical $A_2$.

**9.** Les composés de formule (I) tels que définis à l'une quelconque des revendications 1 à 3, dans lesquels n représente le nombre O.

**10.** Les composés de formule (I) tels que définis à l'une quelconque des revendications 1 à 9, dans lesquels $R_1$ représente un atome d'hydrogène, un atome de chlore, un radical alkyle ou alkényle renfermant jusqu'à 5 atomes de carbone, un radical alkoxy renfermant jusqu'à 5 atomes de carbone ou un radical

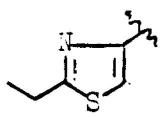 ;

**11.** Les composés de formule (I) tels que définis à l'une quelconque des revendications 1 à 10, dans lesquels $R_2$ représente un atome d'hydrogène.

**12.** Les composés de formule (I) tels que définis à l'une quelconque des revendications 1 à 9, dans lesquels $R_1$ et/ou $R_2$ sont des radicaux aryles éventuellement substitués.

**13.** Les composés de formule (I) tels que définis à l'une quelconque des revendications 1 à 9, dans lesquels $R_1$ est un radical $SCH_3$ et $R_2$ un radical $CH_3$.

**14.** Les composés de formule (I) tels que définis à la revendication 12 ou 13, dans lesquels le groupement alkoxyacrylate est en position 5.

**15.** Les composés de formule (I) tels que définis à la revendication 1, dont les noms suivent :

- le (E,Z) 3-méthoxy [2-méthyl [1-oxo 3-(4-thiazolyl) 2-propényl] amino] 2- propénoate de méthyle,
- le 2-(2-méthylthio 4-méthyl 5-thiazolyl) 3-méthoxy 2-propénoate de méthyle,
- le 2-[N-[3-[2'-éthyl (2,4'-bithiazol) 4-yl] 1-oxo 2-(E)-propényl] méthyl- amino] 3-(Z)-méthoxy propénoate de méthyle,
- le 2-[N-[3-[2'-éthyl (2,4'-bithiazol) 4-yl] 1-oxo 2-(Z)-fluoropropényl] méthylamino] 3-méthoxy (Z) propénoate de méthyle,

- le (Z,Z) 2-[(2-fluoro 1-oxo 3-(4-thiazolyl) 2-propényl) N-méthyl amino) 3- méthoxy 2-propénoate de méthyle,
- le (E,E) alpha-(méthoxy méthylène) 2-[2-(4-thiazolyl) éthényl] benzène acétate de méthyle,
- le 2-(E)-[[[2'-éthyl (2-4'-bithiazol) 4-yl] éthényl] 2-phényl] 3-méthoxy 2-(E)-propénoate de méthyle,
- le (E,Z) 3-méthoxy 2-[méthyl [1-oxo 3-[2-(trifluorométyl) 4-thiazolyl] 2-propényl] amino] 2-propènoate de méthyle.
- l'alpha-(méthoxyméthylène) 2-[(2-phényl 4-thiazolyl) méthoxy] benzène acétate de méthyle,
- le (E) 2-[[2'-éthyl (2,4'-bithiazol)-4-yl] méthoxy] alpha-(méthoxyméthylène) benzène acétate de méthyle,
- l'alpha (E)-(méthoxyméthylène) 2 (E)-[[2-(2-pentyl) 4-thiazolyl] éthényl] benzène acétate de méthyle,
- le (E)-alpha-(méthoxyméthylène) 2-[(4-thiazolyl 2-méthyléthyl] méthoxy] benzène acétate de méthyle,
- le E,E alpha-(méthoxyméthylène) 2-[2-(2-méthyléthyl 4-thiazolyl) éthényl] benzène acétate de méthyle,
- le E,Z alpha-(méthoxyméthylène) 2-[2-(2-méthyléthyl 4-thiazolyl) éthényl] benzène acétate de méthyle,
- l'alpha (Z)-(méthoxyméthylène) 2 (E)-[[2-(2-pentyl) 4-thiazolyl] éthényl] benzène acétate de méthyle,
- l'alpha-(méthoxyméthylène) 2-[(2-méthyl 4-thiazolyl) méthoxy] benzène acétate de méthyle.

**16.** Procédé de préparation des composés de formule (I) tels que définis à la revendication 1, caractérisé en ce que l'on soumet un composé de formule (II) :

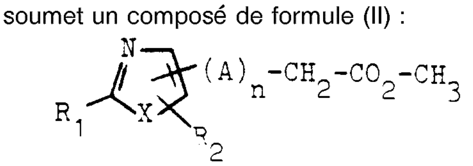

(II)

dans laquelle X, $R_1$, $R_2$, A et n conservent leur signification précédente, à l'action d'une base forte et d'un formiate d'alcoyle ou du N-formylimidazole pour obtenir le composé de formule (III) :

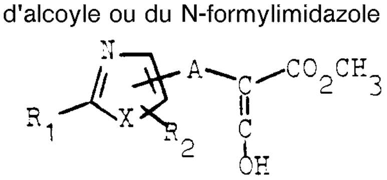

(III)

que l'on soumet à l'action d'un agent capable d'introduire le radical $R_3$ pour obtenir le composé de formule (I) correspondant et sépare le cas échéant les différents isomères.

**17.** Procédé de préparation des composés de formule (I) tels que définis à la revendication 1, dans lesquels le groupe A représente un groupement ($A_1$) tel que défini à la revendication 7, caractérisé en ce que l'on soumet un composé de formule (IV) :

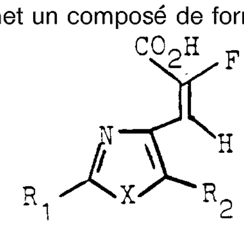

(IV)

dans laquelle X, $R_1$ et $R_2$ conservent leur signification précédente ou un dérivé fonctionnel de ce composé à l'action d'un composé de formule (V) :

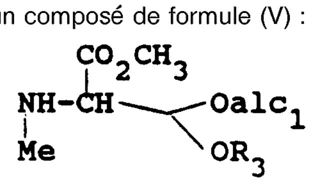

(V)

dans laquelle $R_3$ conserve sa signification précédente et $alc_1$ représente un radical alcoyle renfermant jusqu'à 8 atomes de carbone, pour obtenir le composé de formule (VI) :

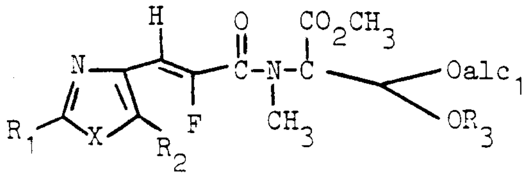

(VI)

que l'on soumet à l'action d'un agent d'élimination du reste de l'alcool $alc_1$-OH, pour obtenir le composé de formule ($I_A$) correspondant :

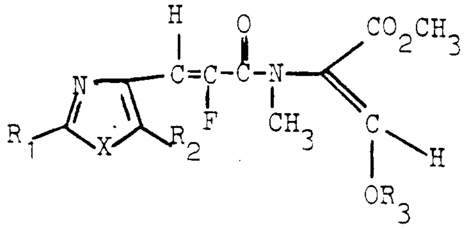

($I_A$)

**18.** Variante du procédé selon la revendication 16 pour préparer les produits de formule (I) dans laquelle A représente un radical

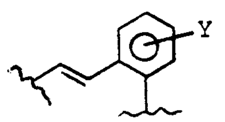

**ou**

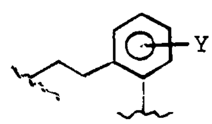

caractérisé en ce que l'on soumet selon la réaction de Wittig-Horner un composé de formule (VII) :

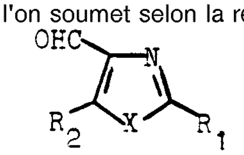

(VII)

dans laquelle X, $R_1$ et $R_2$ conservent leurs significations précédentes à l'action d'un composé de formule (VIII) :

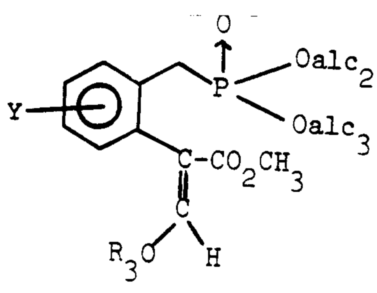

(VIII)

dans laquelle Y et $R_3$ conservent leurs significations précédentes, $alc_2$ et $alc_3$ identiques ou différents représentent un radical alkyle renfermant jusqu'à 8 atomes de carbone, pour obtenir le composé de formule ($I_B$) correspondant :

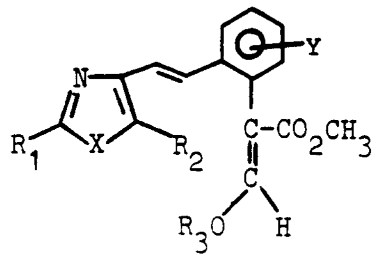

$(I_B)$

que si désiré l'on isole ou si désiré soumet à une hydrogénation catalytique pour obtenir les composés de formule $(I_C)$ :

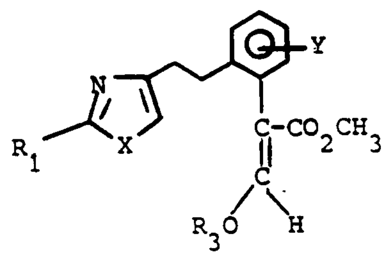

$(I_C)$

**19.** Les composés de formule (II) et (III) définis à la revendication 16, ainsi que les composés (IV) et (VI) définis à la revendication 17.

**20.** Les compositions fongicides renfermant comme principe acitf au moins un composé de formule (I) défini à l'une quelconque des revendications 1 à 14.

**21.** Les compositions fongicides renfermant comme principe actif au moins un composé défini à la revendication 15.

**Revendications pour l'Etat contractant suivant : ES**

**1.** Procédé pour préparer les composés de formule générale (I) :

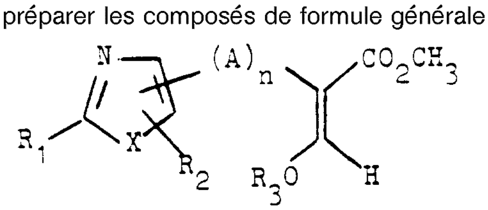

(I)

dans lesquels

- $R_1$ et $R_2$ identiques ou différents représentent un atome d'hydrogène, un atome d'halogène, un radical $CF_3$, un radical alkyle, alkényle, alkynyle ou thioalkyle renfermant jusqu'à 8 atomes de carbone, un radical alkoxy, thioalkoxy ou $SO_2$alkyle renfermant jusqu'à 8 atomes de carbone, un radical aryle, aryloxy ou thioaryle renfermant jusqu'à 18 atomes de carbone éventuellement substitué, un radical hétéroaryle ou hétéroaryloxy éventuellement substitué ou un radical hétérocyclique à 5 ou 6 chaînons éventuellement substitué,
- $R_3$ représente un radical alkyle, alkényle ou alkynyle renfermant jusqu'à 8 atomes de carbone ou un radical aryle renfermant jusqu'à 18 atomes de carbone ou un radical alkoxyalkoxyalkyle renfermant jusqu'à 14 atomes de carbone,
- X représente un atome d'oxygène ou de soufre ou un radical $NR_4$ dans lequel $R_4$ représente un atome d'hydrogène ou un radical alkyle, alkényle ou alkynyle, $SO_2$-alkyle, $SO_2$-alkényle ou $SO_2$ alkynyle renfermant jusqu'à 8 atomes de carbone ou un radical aryle ou $SO_2$-aryle renfermant jusqu'à 18 atomes de carbone éventuellement substitué,
- n représente le nombre O ou le nombre 1,
- A représente un radical choisi dans le groupe des radicaux suivants :

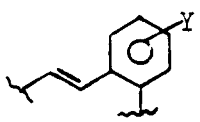 ; 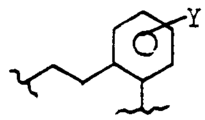 ; 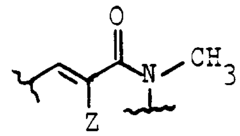 ; 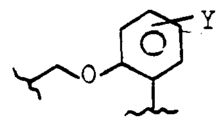

Y représentant un atome d'hydrogène, un atome d'halogene, un radical $CF_3$, un radical alkyle ou alkoxy renfermant juqu'à 8 atomes de carbone et Z représentant un atome d'hydrogène ou un atome d'halogène,

- la géométrie des doubles liaisons étant E ou Z ou un mélange de géométrie E et Z, caractérisé en ce que l'on soumet un composé de formule (II) :

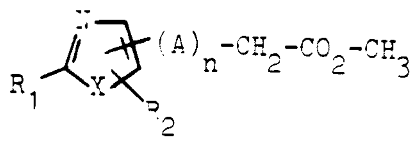

**(II)**

dans laquelle X, $R_1$, $R_2$, A et n conservent leur signification précédente, à l'action d'une base forte et d'un formiate d'alcoyle ou du N-formylimidazole pour obtenir le composé de formule (III) :

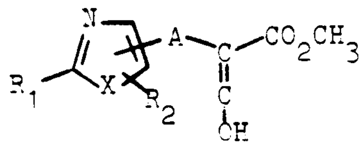

**(III)**

que l'on soumet à l'action d'un agent capable d'introduire le radical $R_3$ pour obtenir le composé de formule (I) correspondant et sépare le cas échéant les différents isomères.

**2.** Procédé pour préparer des produits de formule (I) telle que défini à la revendication 1, caractérisé en ce que :

a) pour préparer des produits répondant à la formule ($I_A$) correspondant à la formule (I) dans laquelle A représente un groupement $A_1$

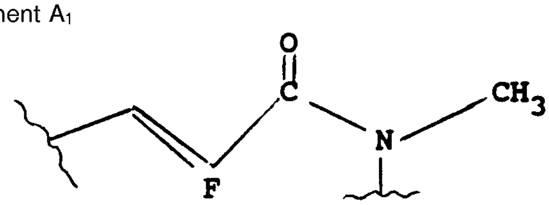

l'on soumet un composé de formule (IV) :

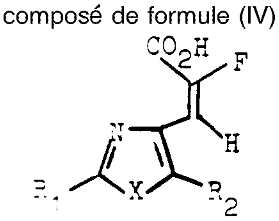

**(IV)**

dans laquelle X, $R_1$ et $R_2$ conservent leur signification précédente ou un dérivé fonctionnel de ce composé à l'action d'un composé de formule (V) :

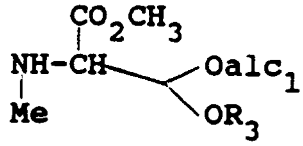

**(V)**

dans laquelle $R_3$ conserve sa signification précédente et $alc_1$ représente un radical alcoyle renfermant jusqu'à 8 atomes de carbone, pour obtenir le composé de formule (VI) :

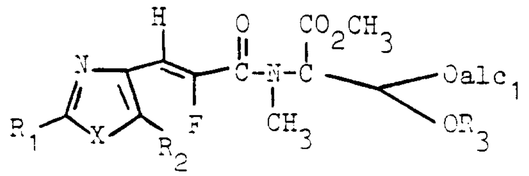

(VI)

que l'on soumet à l'action d'un agent d'élimination du reste de l'alcool $alc_1$-OH, pour obtenir le composé de formule ($I_A$) correspondant :

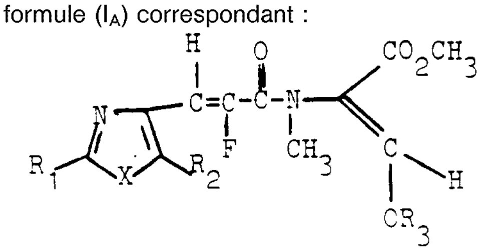

($I_A$)

et sépare le cas échéant les isomères au niveau de la double liaison ;

b) pour préparer des produits répondant à la formule ($I_B$) correspondant à la formule (I) dans laquelle A représente un groupement

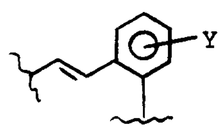

ou répondant à la formule ($I_C$) correspondant à la formule (I) dans laquelle A représente un groupement

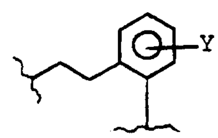

l'on soumet selon la réaction de Wittig-Horner un composé de formule (VII) :

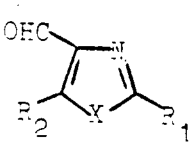

(VII)

dans laquelle X, $R_1$ et $R_2$ conservent leurs significations précédentes à l'action d'un composé de formule (VIII) :

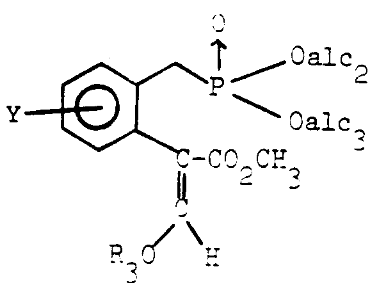

(VIII)

dans laquelle Y et $R_3$ conservent leurs significations précédentes, $alc_2$ et $alc_3$ identiques ou différents représentent un radical alkyle renfermant jusqu'à 8 atomes de carbone, pour obtenir le

composé de formule ($I_B$) correspondant :

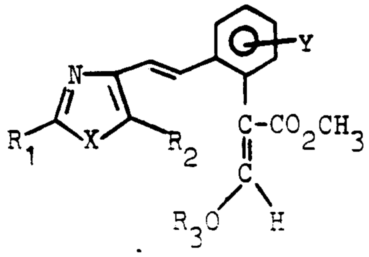

($I_B$)

que si désiré l'on isole ou si désiré soumet à une hydrogénation catalytique pour obtenir les composés de formule ($I_C$) :

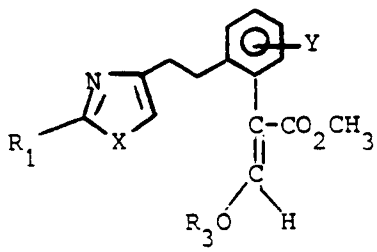

($I_C$)

et sépare le cas échéant les isomères au niveau de la double liaison.

**3.** Procédé selon la revendication 2 pour préparer des composés de formule ($I_A$) telle que définie à la revendication 2, caractérisé en ce que l'on soumet un composé de formule (IV) telle que définie à la revendication 2 ou un dérivé fonctionnel de ce composé à l'action d'un composé de formule (V) telle que définie à la revendication 2 pour obtenir le composé de formule (VI) que l'on soumet à l'action d'un agent d'élimination de l'alcool $alc_1$-OH pour obtenir le composé de formule ($I_A$) correspondant et sépare le cas échéant les isomères au niveau de la double liaison.

**4.** Procédé selon la revendication 2 pour préparer des composés de formule ($I_B$) ou ($I_C$) telle que définie à la revendication 2, caractérisé en ce que l'on soumet selon la réaction de Wittig-Horner un composé de formule (VII) telle que définie à la revendication 2 à l'action d'un composé de formule (VIII) telle que définie à la revendication 2 pour obtenir le composé de formule ($I_B$) que si désiré l'on isole et le cas échéant sépare les isomères au niveau de la double liaison ou si désiré soumet à une hydrogénation catalytique pour obtenir le composé de formule ($I_C$) et le cas échéant sépare les isomères au niveau de la double liaison.

**5.** Procédé selon la revendication 1 ou 2, caractérisé en ce que l'on utilise au départ un composé de formule (II), (IV) ou (VII) dans laquelle X représente un atome de soufre.

**6.** Procédé selon la revendication 2 ou 4, caractérisé en ce que l'on utilise au départ un composé de formule (VIII) dans laquelle Y représente un atome d'hydrogène.

**7.** Procédé selon la revendication 1, caractérisé en ce que l'on utilise au départ un composé de formule (II) dans laquelle A représente un radical :

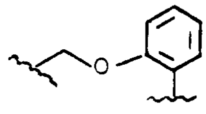

**8.** Procédé selon la revendication 1, caractérisé en ce que l'on utilise au départ un composé de formule (II) dans laquelle A représente un radical :

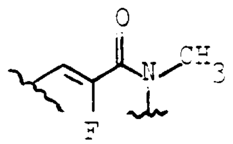

(A1)

ou

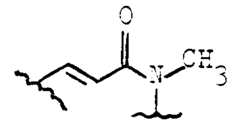

(A2)

**9.** Procédé selon la revendication 1, caractérisé en ce que l'on utilise au départ un composé de formule (II) dans laquelle n représente le nombre O.

**10.** Procédé selon l'une quelconque des revendications 1 à 9, caractérisé en ce que l'on utilise au départ un composé de formule (II), (IV) ou (VII) dans laquelle $R_1$ représente un atome d'hydrogène, un atome de chlore, un radical alkyle ou alkényle renfermant jusqu'à 5 atomes de carbone, un radical alkoxy renfermant jusqu'à 5 atomes de carbone ou un radical

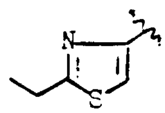 .

**11.** Procédé selon l'une quelconque des revendications 1 à 9, caractérisé en ce que l'on utilise au départ un composé de formule (II), (IV) ou (VII) dans laquelle $R_2$ représente un atome d'hydrogène.

**12.** Procédé selon l'une quelconque des revendications 1 à 9, caractérisé en ce que l'on utilise au départ un composé de formule (II), (IV) ou (VII) dans laquelle $R_1$ et/ou $R_2$ sont des radicaux aryles éventuellement substitués.

**13.** Procédé selon l'une quelconque des revendications 1 à 9, caractérisé en ce que l'on utilise au départ un composé de formule (II), (IV) ou (VII) dans laquelle $R_1$ est un radical $SCH_3$ et $R_2$ un radical $CH_3$.

**14.** Procédé selon la revendication 12 ou 13, caractérisé en ce que l'on utilise au départ un composé de formule (II) le groupement alkoxyacrylate est en position 5.

**15.** Procédé selon la revendication 1 ou 2, caractérisé en ce que l'on prépare l'un quelconque des produits dont les noms suivent :

- le (E,Z) 3-méthoxy [2-méthyl [1-oxo 3-(4-thiazolyl) 2-propényl] amino] 2- propénoate de méthyle,
- le 2-(2-méthylthio 4-méthyl 5-thiazolyl) 3-méthoxy 2-propénoate de méthyle,
- le 2-[N-[3-[2'-éthyl (2,4'-bithiazol) 4-yl] 1-oxo 2-(E)-propényl] méthyl- amino] 3-(Z)-méthoxy propénoate de méthyle,
- le 2-[N-[3-[2'-éthyl (2,4'-bithiazol) 4-yl] 1-oxo 2-(Z)-fluoropropényl] méthylamino] 3-méthoxy (Z) propénoate de méthyle,
- le (Z,Z) 2-[(2-fluoro 1-oxo 3-(4-thiazolyl) 2-propényl) N-méthyl amino] 3- méthoxy 2-propénoate de méthyle,
- le (E,E) alpha-(méthoxy méthylène) 2-[2-(4-thiazolyl) éthényl] benzène acétate de méthyle,
- le 2-(E)-[[[2'-éthyl (2,4'-bithiazol) 4-yl] éthényl] 2-phényl] 3-méthoxy 2-(E)-propénoate de méthyle,
- le (E,Z) 3-méthoxy 2-[méthyl [1-oxo 3-[2-(trifluorométyl) 4-thiazolyl] 2-propényl] amino] 2-propènoate de méthyle.
- l'alpha-(méthoxyméthylène) 2-[(2-phényl 4-thiazolyl) méthoxy] benzène acétate de méthyle,
- le (E) 2-[[2'-éthyl (2,4'-bithiazol)-4-yl] méthoxy] alpha-(méthoxyméthylène) benzène acétate de méthyle,
- l'alpha (E)-(méthoxyméthylène) 2 (E)-[[2-(2-pentyl) 4-thiazolyl] éthényl] benzène acétate de méthyle,
- le (E)-alpha-(méthoxyméthylène) 2-[(4-thiazolyl 2-méthyléthyl] méthoxy] benzène acétate de méthyle,
- le E,E alpha-(méthoxyméthylène) 2-[2-(2-méthyléthyl 4-thiazolyl) éthényl] benzène acétate de méthyle,

- le E,Z alpha-(méthoxyméthylène) 2-[2-(2-méthyléthyl 4-thiazolyl) éthényl] benzène acétate de méthyle,
- l'alpha (Z)-(méthoxyméthylène) 2 (E)-[[2-(2-pentyl) 4-thiazolyl] éthényl] benzène acétate de méthyle,
- l'alpha-(méthoxyméthylène) 2-[(2-méthyl 4-thiazolyl) méthoxy] benzène acétate de méthyle.

**16.** Procédé de préparation de compositions fongicides, caractérisé en ce que l'on met à titre de principe actif au moins un composé de formule (I) tel que défini à l'une quelconque des revendications 1 à 14.

**17.** Procédé de préparation de compositions fongicides, caractérisé en ce que l'on met à titre de principe actif au moins un composé de formule (I) tel que défini à la revendication 15.

**Claims**

**Claims for the following Contracting States : AT, BE, CH, DE, DK, FR, GB, GR, IT, LI, LU, NL, SE**

**1.** The compounds of general formula (I):

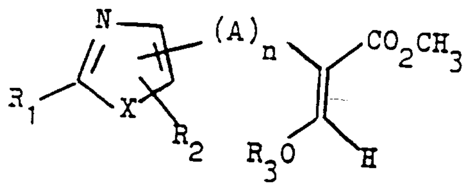

(I)

in which

- $R_1$ and $R_2$, identical or different, represent a hydrogen atom, a halogen atom, a $CF_3$ radical, an alkyl, alkenyl, alkynyl or thioalkyl radical containing up to 8 carbon atoms, an alkoxy, thioalkoxy or $SO_2$alkyl radical containing up to 8 carbon atoms, an optionally substituted aryl, aryloxy or thioaryl radical containing up to 18 carbon atoms, an optionally substituted heteroaryl or heteroaryloxy radical or an optionally substituted heterocyclic radical with 5 or 6 members,
- $R_3$ represents an alkyl, alkenyl or alkynyl radical containing up to 8 carbon atoms or an aryl radical containing up to 18 carbon atoms or an alkoxyalkoxyalkyl radical containing up to 14 carbon atoms,
- X represents an oxygen or sulphur atom or an $NR_4$ radical in which $R_4$ represents a hydrogen atom or an alkyl, alkenyl or alkynyl, $SO_2$-alkyl, $SO_2$-alkenyl or $SO_2$-alkynyl radical containing up to 8 carbon atoms or an aryl or $SO_2$-aryl radical containing up to 18 carbon atoms, optionally substituted,
- n represents the number 0 or the number 1,
- A represents a radical chosen from the following group of radicals:

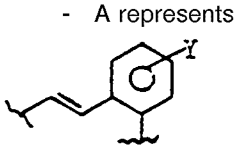 ; 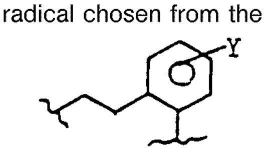 ; 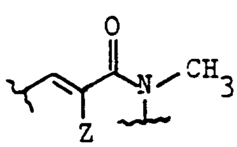 ; 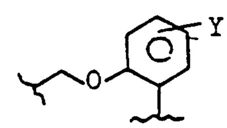

Y representing a hydrogen atom, a halogen atom, a $CF_3$radical, an alkyl or alkoxy radical containing up to 8 carbon atoms and Z representing a hydrogen atom or a halogen atom,
- the geometry of the double bonds being E or Z or a mixture of E and Z geometry.

**2.** The compounds of formula (I) as defined in claim 1, in which the group

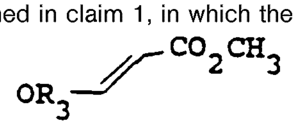

is such that $CO_2CH_3$ and $OR_3$ are of trans configuration relative to each other.

**3.** The compounds of formula (I) as defined in claim 1 or 2 in which X represents a sulphur atom.

4. The compounds of formula (I) as defined in any one of claims 1 to 3 in which A represents a radical

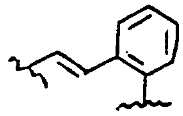

5. The compounds of formula (I) as defined in claim 4, in which the geometry of the double bond is E.

6. The compounds of formula (I) as defined in any one of claims 1 to 3, in which A represents a radical

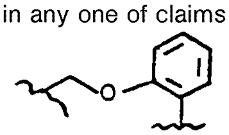

7. The compounds of formula (I) as defined in any one of claims 1 to 3, in which A represents a radical

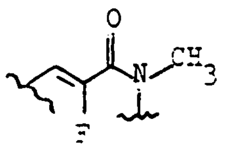

($A_1$)

or

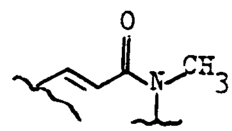

8. The compounds of formula (I) as defined in claim 7, in which the geometry of the double bond is Z for the $A_1$ radical and E for the $A_2$ radical.

9. The compounds of formula (I) as defined in any one of claims 1 to 3, in which n represents the number 0.

10. The compounds of formula (I) as defined in any one of claims 1 to 9, in which $R_1$ represents a hydrogen atom, a chlorine atom, an alkyl or alkenyl radical containing up to 5 carbon atoms, an alkoxy radical containing up to 5 carbon atoms or a radical

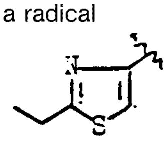 ;

11. The compounds of formula (I) as defined in any one of claims 1 to 10, in which $R_2$ represents a hydrogen atom.

12. The compounds of formula (I) as defined in any one of claims 1 to 9, in which $R_1$ and/or $R_2$ are optionally substituted aryl radicals.

13. The compounds of formula (I) as defined in any one of claims 1 to 9, in which $R_1$ is an $SCH_3$ radical and $R_2$ is a $CH_3$ radical.

14. The compounds of formula (I) as defined in claim 12 or 13, in which the alkoxyacrylate group is in position 5.

15. The compounds of formula (I) as defined in claim 1, of which the names follow:
- methyl (E,Z) 3-methoxy [2-methyl [1-oxo 3-(4-thiazolyl) 2-propenyl] amino] 2-propenoate,
- methyl 2-(2-methylthio 4-methyl 5-thiazolyl) 3-methoxy 2-propenoate,

- methyl 2-[N-[3-[2'-ethyl (2,4'-bithiazol) 4-yl] 1-oxo 2-(E)-propenyl] methylamino] 3-(Z)-methoxy propenoate,
- methyl 2-[N-[3-[2'-ethyl (2,4'-bithiazol) 4-yl] 1-oxo 2-(Z)-fluoropropenyl] methylamino] 3-methoxy (Z) propenoate,
- methyl (Z,Z) 2-[(2-fluoro 1-oxo 3-(4-thiazolyl) 2-propenyl) N-methyl amino] 3-methoxy 2-propenoate,
- methyl (E,E) alpha-(methoxy methylene) 2-[2-(4-thiazolyl) ethenyl] benzene acetate,
- methyl 2-(E)-[[[2'-ethyl (2,4'-bithiazol) 4-yl] ethenyl] 2-phenyl] 3-methoxy 2-(E)-propenoate,
- methyl (E,Z) 3-methoxy 2-[methyl [1-oxo 3-[2-(trifluoromethyl) 4-thiazolyl] 2-propenyl] amino] 2-propenoate,
- methyl alpha-(methoxymethylene) 2-[(2-phenyl 4-thiazolyl) methoxy] benzene acetate,
- methyl (E) 2-[[2'-ethyl (2,4'-bithiazol)-4-yl] methoxy] alpha-(methoxymethylene) benzene acetate,
- methyl alpha (E)-(methoxymethylene) 2 (E)-[[2-(2-pentyl) 4-thiazolyl] ethenyl] benzene acetate,
- methyl (E)-alpha-(methoxymethylene) 2-[(4-thiazolyl 2-methylethyl] methoxy] benzene acetate,
- methyl E,E alpha-(methoxymethylene) 2-[2-(2-methylethyl 4-thiazolyl) ethenyl] benzene acetate,
- methyl E,Z alpha-(methoxymethylene) 2-[2-(2-methylethyl 4-thiazolyl) ethenyl] benzene acetate,
- methyl alpha (Z)-(methoxymethylene) 2 (E)-[[2-(2-pentyl) 4-thiazolyl] ethenyl] benzene acetate,
- methyl alpha-(methoxymethylene) 2-[(2-methyl 4-thiazolyl) methoxy] benzene acetate.

**16.** Preparation process for compounds of formula (I) as defined in claim 1, characterized in that a compound of formula (II):

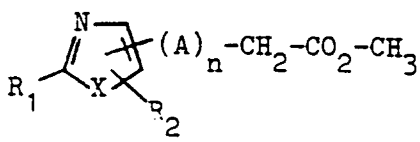

(II)

in which X, $R_1$, $R_2$, A and n keep their previous meaning, is subjected to the action of a strong base and an alkyl formate or N-formylimidazole in order to obtain the compound of formula (III):

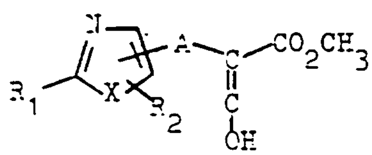

(III)

which is subjected to the action of an agent capable of introducing the $R_3$ radical in order to obtain the corresponding compound of formula (I) and if appropriate the different isomers are separated.

**17.** Preparation process for compounds of formula (I) as defined in claim 1, in which group A represents an ($A_1$) group as defined in claim 7, characterized in that a compound of formula (IV):

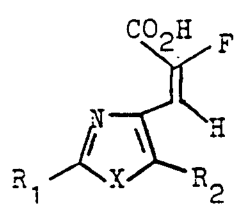

(IV)

in which X, $R_1$ and $R_2$ keep their previous meaning, or a functional derivative of this compound, is subjected to the action of a compound of formula (V):

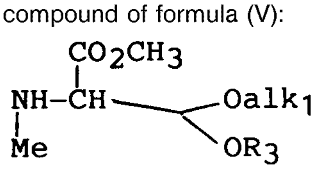

(V)

in which $R_3$ keeps its previous meaning and $alk_1$ represents an alkyl radical containing up to 8 carbon

atoms, in order to obtain the compound of formula (VI):

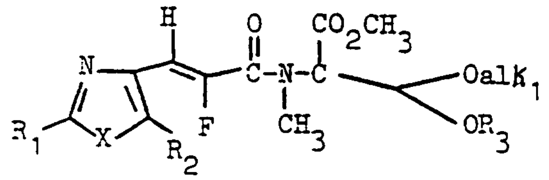

(VI)

which is subjected to the action of an elimination agent of the remainder of the $alk_1$-OH alcohol, in order to obtain the corresponding compound of formula ($I_A$):

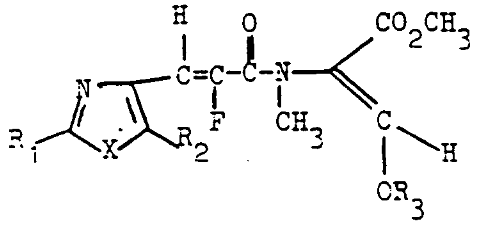

($I_A$)

**18.** Variant of the process according to claim 16 for preparing the products of formula (I) in which A represents a radical

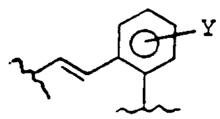

or

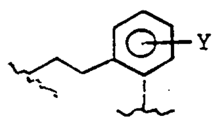

characterized in that, according to the Wittig-Horner reaction, a compound of formula (VII):

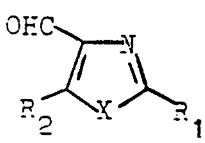

(VII)

in which X, $R_1$ and $R_2$ keep their previous meanings, is subjected to the action of a compound of formula (VIII):

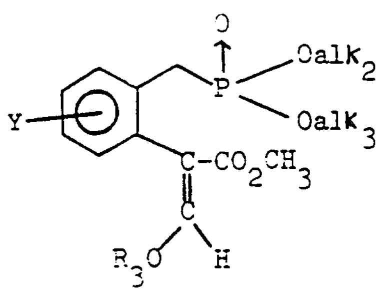

(VIII)

in which Y and $R_3$ keep their previous meanings, $alk_2$ and $alk_3$, identical or different, represent an alkyl radical containing up to 8 carbon atoms, in order to obtain the corresponding compound of formula ($I_B$):

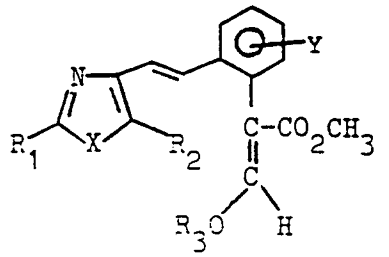

$(I_B)$

which if desired is isolated or is desired is subjected to a catalytic hydrogenation in order to obtain the compounds of formula ($I_C$):

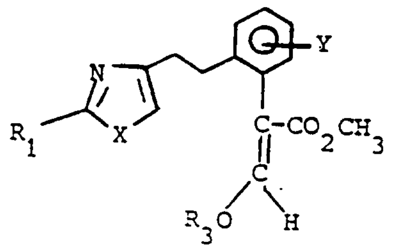

$(I_C)$

**19.** The compounds of formula (II) and (III) defined in claim 16, as well as the compounds (IV) and (VI) defined in claim 17.

**20.** The fungicide compositions containing as active ingredient at least one compound of formula (I) defined in any one of claims 1 to 14.

**21.** The fungicide compositions containing as active ingredient at least one compound defined in claim 15.

**Claims for the following Contracting State : ES**

**1.** Process for preparing the compounds of general formula (I):

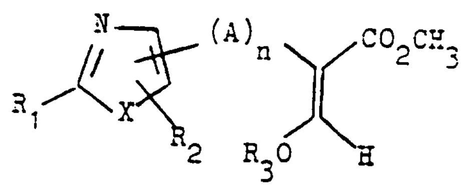

(I)

in which

- $R_1$ and $R_2$, identical or different, represent a hydrogen atom, a halogen atom, a $CF_3$ radical, an alkyl, alkenyl, alkynyl or thioalkyl radical containing up to 8 carbon atoms, an alkoxy, thioalkoxy or $SO_2$alkyl radical containing up to 8 carbon atoms, an optionally substituted aryl, aryloxy or thioaryl radical containing up to 18 carbon atoms, an optionally substituted heteroaryl or heteroaryloxy radical or an optionally substituted heterocyclic radical with 5 or 6 members,
- $R_3$ represents an alkyl, alkenyl or alkynyl radical containing up to 8 carbon atoms or an aryl radical containing up to 18 carbon atoms or an alkoxyalkoxyalkyl radical containing up to 14 carbon atoms,
- X represents an oxygen or sulphur atom or an $NR_4$ radical in which $R_4$ represents a hydrogen atom or an alkyl, alkenyl or alkynyl, $SO_2$-alkyl, $SO_2$-alkenyl or $SO_2$-alkynyl radical containing up to 8 carbon atoms or an aryl or $SO_2$-aryl radical containing up to 18 carbon atoms, optionally substituted,
- n represents the number 0 or the number 1,
- A represents a radical chosen from the following group of radicals:

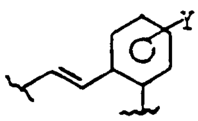 ; 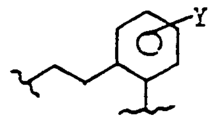 ; 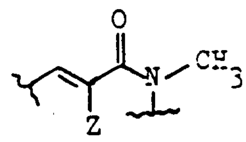 ; 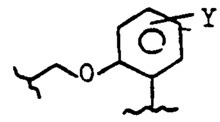

Y representing a hydrogen atom, a halogen atom, a $CF_3$ radical, an alkyl or alkoxy radical containing up to 8 carbon atoms and Z representing a hydrogen atom or a halogen atom,

- the geometry of the double bonds being E or Z or a mixture of E and Z geometry, characterized in that a compound of formula (II):

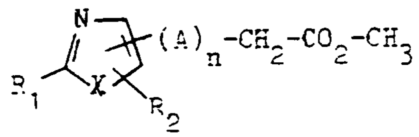

(II)

in which X, $R_1$, $R_2$, A and n keep their previous meaning, is subjected to the action of a strong base and an alkyl formate or N-formylimidazole in order to obtain the compound of formula (III):

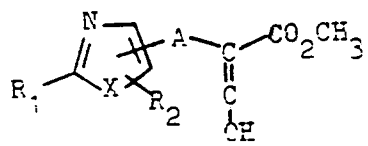

(III)

which is subjected to the action of an agent capable of introducing the $R_3$ radical in order to obtain the corresponding compound of formula (I) and if appropriate the different isomers are separated.

2. Process for preparing products of formula (I) as defined in claim 1, characterized in that:
a) to prepare products corresponding to formula ($I_A$) which corresponds to formula (I) in which A represents an $A_1$ group

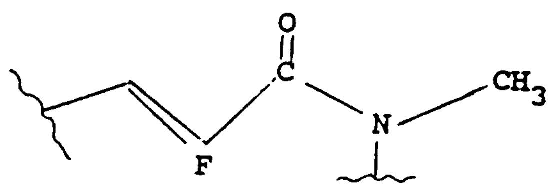

a compound of formula (IV):

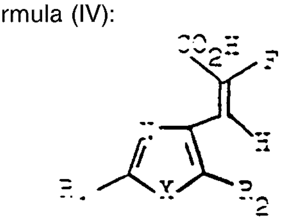

(IV)

in which X, $R_1$ and $R_2$ keep their previous meaning, or a functional derivative of this compound, is subjected to the action of a compound of formula (V):

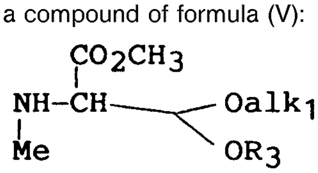

(V)

in which $R_3$ keeps its previous meaning and $alk_1$ represents an alkyl radical containing up to 8 carbon atoms, in order to obtain the compound of formula (VI):

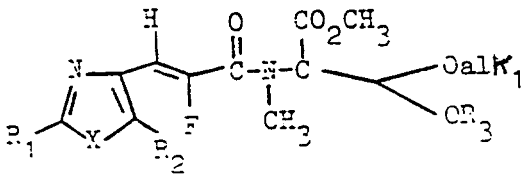

(VI)

which is subjected to the action of an elimination agent of the remainder of the $alk_1$-OH alcohol, in order to obtain the corresponding compound of formula ($I_A$):

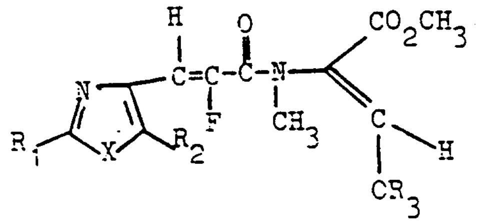

($I_A$)

and if appropriate the isomers are separated at the double bond;

b) to prepare products corresponding to formula ($I_B$) which corresponds to formula (I) in which A represents a

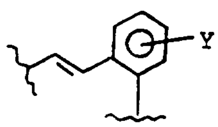

group or corresponding to formula ($I_C$) which corresponds to formula (I) in which A represents a

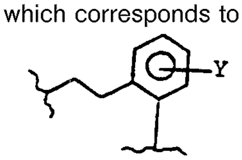

group, according to the Wittig-Horner reaction, a compound of formula (VII):

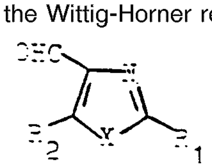

(VII)

in which X, $R_1$ and $R_2$ keep their previous meanings, is subjected to the action of a compound of formula (VIII):

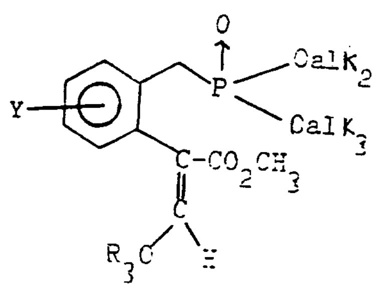

(VIII)

in which Y and $R_3$ keep their previous meanings, $alk_2$ and $alk_3$, identical or different, represent an

alkyl radical containing up to 8 carbon atoms, in order to obtain the corresponding compound of formula ($I_B$):

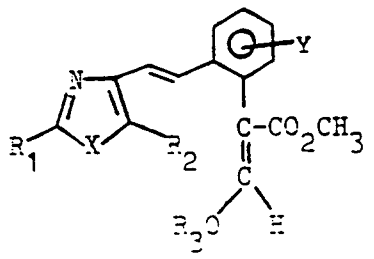

($I_B$)

which if desired is isolated or is desired is subjected to a catalytic hydrogenation in order to obtain the compounds of formula ($I_C$):

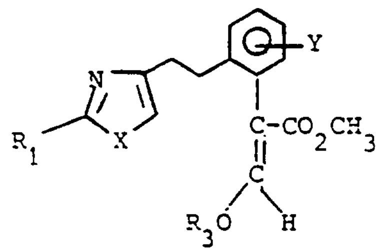

($I_C$)

and if appropriate the isomers are separated at the double bond.

3. Process according to claim 2 for preparing compounds of formula ($I_A$) as defined in claim 2, characterized in that a compound of formula (IV) as defined in claim 2 or a functional derivative of this compound is subjected to the action of a compound of formula (V) as defined in claim 2 in order to obtain the compound of formula (VI) which is subjected to the action of an elimination agent of the $alk_1$-OH alcohol in order to obtain the corresponding compound of formula ($I_A$) and if appropriate the isomers are separated at the double bond.

4. Process according to claim 2 for preparing compounds of formula ($I_B$) or ($I_C$) as defined in claim 2, characterized in that according to the Wittig-Horner reaction, a compound of formula (VII) as defined in claim 2 is subjected to the action of a compound of formula (VIII) as defined in claim 2 in order to obtain the compound of formula ($I_B$) which if desired is isolated and if appropriate the isomers are separated at the double bond, or if desired is subjected to a catalytic hydrogenation in order to obtain the compound of formula ($I_C$) and if appropriate the isomers are separated at the double bond.

5. Process according to claim 1 or 2, characterized in that a compound of formula (II), (IV) or (VII) is used at the start in which X represents a sulphur atom.

6. Process according to claim 2 or 4, characterized in that a compound of formula (VIII) is used at the start in which Y represents a hydrogen atom.

7. Process according to claim 1, characterized in that a compound of formula (II) is used at the start in which A represents a radical:

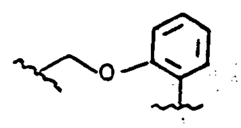

8. Process according to claim 1, characterized in that a compound of formula (II) is used at the start in which A represents a radical:

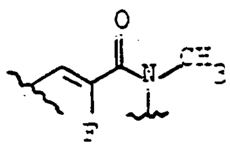 or 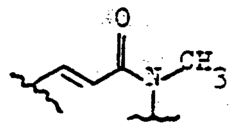

(A₁) (A₂)

9. Process according to claim 1, characterized in that a compound of formula (II) is used at the start in which n represents the number 0.

10. Process according to any one of claims 1 to 9, characterized in that a compound of formula (II), (IV) or (VII) is used at the start in which $R_1$ represents a hydrogen atom, a chlorine atom, an alkyl or alkenyl radical containing up to 5 carbon atoms, an alkoxy radical containing up to 5 carbon atoms or a

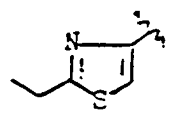

radical.

11. Process according to any one of claims 1 to 9, characterized in that a compound of formula (II), (IV) or (VII) is used at the start in which $R_2$ represents a hydrogen atom.

12. Process according to any one of claims 1 to 9, characterized in that a compound of formula (II), (IV) or (VII) is used at the start in which $R_1$ and/or $R_2$ are optionally substituted aryl radicals.

13. Process according to any one of claims 1 to 9, characterized in that a compound of formula (II), (IV) or (VII) is used at the start in which $R_1$ is an $SCH_3$ radical and $R_2$ is a $CH_3$ radical.

14. Process according to claim 12 or 13, characterized in that a compound of formula (II) is used at the start in which the alkoxyacrylate group is in position 5.

15. Process according to claim 1 or 2, characterized in that any one of the products is prepared of which the names follow:
- methyl (E,Z) 3-methoxy [2-methyl [1-oxo 3-(4-thiazolyl) 2-propenyl] amino] 2-propenoate,
- methyl 2-(2-methylthio 4-methyl 5-thiazolyl) 3-methoxy 2-propenoate,
- methyl 2-[N-[3-[2'-ethyl (2,4'-bithiazol) 4-yl] 1-oxo 2-(E)-propenyl] methylamino] 3-(Z)-methoxy propenoate,
- methyl 2-[N-[3-[2'-ethyl (2,4'-bithiazol) 4-yl] 1-oxo 2-(Z)-fluoropropenyl] methylamino] 3-methoxy (Z) propenoate,
- methyl (Z,Z) 2-[(2-fluoro 1-oxo 3-(4-thiazolyl) 2-propenyl) N-methyl amino] 3-methoxy 2-propenoate,
- methyl (E,E) alpha-(methoxy methylene) 2-[2-(4-thiazolyl) ethenyl] benzene acetate,
- methyl 2-(E)-[[[2'-ethyl (2,4'-bithiazol) 4-yl] ethenyl] 2-phenyl] 3-methoxy 2-(E)-propenoate,
- methyl (E,Z) 3-methoxy 2-[methyl [1-oxo 3-[2-(trifluoromethyl) 4-thiazolyl] 2-propenyl] amino] 2-propenoate,
- methyl alpha-(methoxymethylene) 2-[(2-phenyl 4-thiazolyl) methoxy] benzene acetate,
- methyl (E) 2-[[2'-ethyl (2,4'-bithiazol)-4-yl] methoxy] alpha-(methoxymethylene) benzene acetate,
- methyl alpha (E)-(methoxymethylene) 2 (E)-[[2-(2-pentyl) 4-thiazolyl] ethenyl] benzene acetate,
- methyl (E)-alpha-(methoxymethylene) 2-[(4-thiazolyl 2-methylethyl] methoxy] benzene acetate,
- methyl E,E alpha-(methoxymethylene) 2-[2-(2-methylethyl 4-thiazolyl) ethenyl] benzene acetate,
- methyl E,Z alpha-(methoxymethylene) 2-[2-(2-methylethyl 4-thiazolyl) ethenyl] benzene acetate,
- methyl alpha (Z)-(methoxymethylene) 2 (E)-[[2-(2-pentyl) 4-thiazolyl] ethenyl] benzene acetate,
- methyl alpha-(methoxymethylene) 2-[(2-methyl 4-thiazolyl) methoxy] benzene acetate.

16. Preparation process for fungicide compositions, characterized in that at least one compound of formula (I) as defined in any one of claims 1 to 14 is used as active ingredient.

17. Preparation process for fungicide compositions, characterized in that at least one compound of formula (I) as defined in claim 15 is used as active ingredient.

**Patentansprüche**

**Patentansprüche für folgende Vertragsstaaten : AT, BE, CH, DE, DK, FR, GB, GR, IT, LI, LU, NL, SE**

1. Verbindungen der allgemeinen Formel (I)

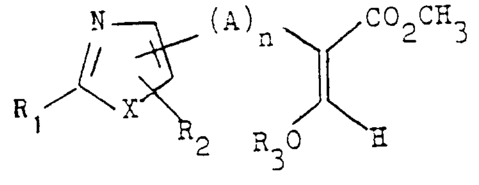

worin

- $R_1$ und $R_2$, die gleich oder verschieden sind, für ein Wasserstoffatom, ein Halogenatom, eine $CF_3$-Gruppe, einen Alkyl-, Alkenyl-, Alkinyl- oder Thioalkylrest mit bis zu 8 Kohlenstoffatomen, einen Alkoxy-, Thioalkoxy- oder $SO_2$-Alkylrest mit bis zu 8 Kohlenstoffatomen, einen Aryl-, Aryloxy- oder Thioarylrest mit bis zu 18 Kohlenstoffatomen, der gegebenenfalls substituiert ist, einen Heteroaryl- oder Heteroaryloxyrest, der gegebenenfalls substituiert ist, oder einen heterocyclischen, 5- oder 6-gliedrigen, gegebenenfalls substituierten Rest stehen,
- $R_3$ für einen Alkyl-, Alkenyl- oder Alkinylrest mit bis zu 8 Kohlenstoffatomen oder einen Arylrest mit bis zu 18 Kohlenstoffatomen oder einen Alkoxyalkoxyalkylrest mit bis zu 14 Kohlenstoffatomen steht,
- X für ein Sauerstoff- oder Schwefelatom oder einen $NR_4$-Rest steht, worin $R_4$ für ein Wasserstoffatom oder einen Alkyl-, Alkenyl- oder Alkinylrest, einen $SO_2$-Alkyl-, $SO_2$-Alkenyl- oder $SO_2$-Alkinylrest mit bis zu 8 Kohlenstoffatomen oder einen Aryl- oder $SO_2$-Arylrest mit bis zu 18 Kohlenstoffatomen, der gegebenenfalls substituiert ist, steht,
- n den Wert 0 oder 1 hat,
- A für einen Rest ausgewählt aus der Gruppe der folgenden Reste

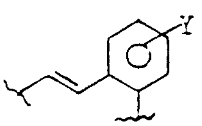 ; 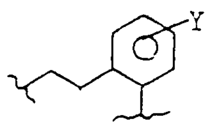 ; 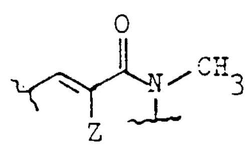 ; 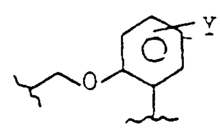

worin Y für ein Wasserstoffatom, ein Halogenatom, eine $CF_3$-Gruppe, einen Alkyl- oder Alkoxyrest mit bis zu 8 Kohlenstoffatomen steht, und Z für ein Wasserstoffatom oder ein Halogenatom steht, steht,

- die Geometrie der Doppelbindungen E oder Z oder ein Gemisch aus der Geometrie E und Z ist.

2. Verbindungen der Formel (I) nach Anspruch 1, worin die Gruppierung

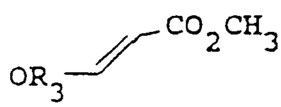

so ist, daß $CO_2CH_3$ und $OR_3$ in Trans-Konformation zueinander stehen.

3. Verbindungen der Formel (I) nach Anspruch 1 oder 2, worin X für ein Schwefelatom steht.

4. Verbindungen der Formel (I) nach einem der Ansprüche 1 bis 3, worin A für einen Rest

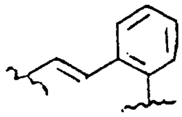

steht.

5. Verbindungen der Formel (I) nach Anspruch 4, worin die Geometrie der Doppelbindung E ist.

6. Verbindungen der Formel (I) nach einem der Ansprüche 1 bis 3, worin A für einen Rest

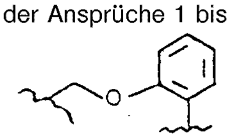

steht.

7. Verbindungen der Formel (I) nach einem der Ansprüche 1 bis 3, worin A für einen Rest

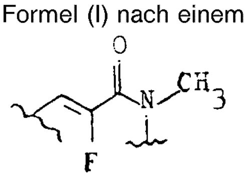

(A₁)

oder

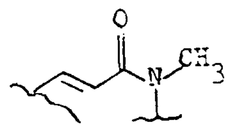

(A₂)

steht.

8. Verbindungen der Formel (I) nach Anspruch 7, worin die Geometrie der Doppelbindung für den Rest $A_1$ Z und für den Rest $A_2$ E ist.

9. Verbindungen der Formel (I) nach einem der Ansprüche 1 bis 3, worin n den Wert 0 besitzt.

10. Verbindungen der Formel (I) nach einem der Ansprüche 1 bis 9, worin $R_1$ für ein Wasserstoffatom, ein Chloratom, einen Alkyl- oder Alkenylrest mit bis zu 5 Kohlenstoffatomen, einen Alkoxyrest mit bis zu 5 Kohlenstoffatomen oder einen Rest

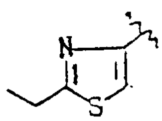 ;

steht.

11. Verbindungen der Formel (I) nach einem der Ansprüche 1 bis 10, worin $R_2$ für ein Wasserstoffatom steht.

12. Verbindungen der Formel (I) nach einem der Ansprüche 1 bis 9, worin $R_1$ und/oder $R_2$ gegebenenfalls substituierte Arylreste sind.

13. Verbindungen der Formel (I) nach einem der Ansprüche 1 bis 9, worin $R_1$ eine $SCH_3$-Gruppe ist, und $R_2$ eine $CH_3$-Gruppe ist.

14. Verbindungen der Formel (I) nach Anspruch 12 oder 13, worin die Alkoxyacrylatgruppierung in Position 5 steht.

15. Verbindungen der Formel (I) nach Anspruch 1, nämlich

- (E,Z)-3-Methoxy-[2-methyl-[1-oxo-3-(4-thiazolyl)-2-propenyl]amino]-2-methylpropenoat,
- 2-(2-Methylthio-4-methyl-5-thiazolyl)-3-methoxy-2-methylpropenoat,
- 2-[N-[3-[2'-ethyl-(2,4'-bisthiazol)-4-yl]-1-oxo-2-(E)-propenyl]methylamino]-3-(Z)-methoxymethylpropenoat,
- 2-[N-[3-[2'-ethyl-(2,4'-bisthiazol)-4-yl]-1-oxo-2-(Z)-fluorpropenyl]methylamino]-3-methoxy-(Z)-methylpropenoat,
- (Z,Z)-2-[(2-Fluor-1-oxo-3-(4-thiazolyl)-2-propenyl)-N-methylamino]-3-methoxy-2-methylpropenoat,
- (E,E)-α-(Methoxymethylen)-2-[2-(4-thiazolyl)ethenyl]benzylmethylacetat,
- 2-(E)-[[[2'-ethyl-(2,4'-bisthiazol)-4-yl]ethenyl]-2-phenyl]-3-methoxy-2-(E)-methylpropenoat,
- (E,Z)-3-Methoxy-2-[methyl-[1-oxo-3-[2-(trifluormethyl)-4-thiazolyl]-2-propenyl]amino]-2-methylpropenoat,
- α-(Methoxymethylen)-2-[(2-phenyl-4-thiazolyl)methoxy] benzylmethylacetat,
- (E)-2-[[2'-ethyl-(2,4'-bisthiazol)-4-yl]methoxy]-α-(methoxymethylen)benzylmethylacetat,
- α-(E)-(Methoxymethylen)-2-(E)-[[2-(2-pentyl)-4-thiazolyl]ethenyl]benzylmethylacetat,
- (E)-α-(Methoxymethylen)-2-[(4-thiazolyl-2-methylethyl]methoxy]benzylmethylacetat,
- E,E-α-(Methoxymethylen)-2-[2-(2-methylethyl-4-thiazolyl)ethenyl]benzylmethylacetat,
- E,Z-α-(Methoxymethylen)-2-[2-(2-methylethyl-4-thiazolyl)ethenyl]benzylmethylacetat,
- α-(Z)-(Methoxymethylen)-2-(E)-[[2-(2-pentyl)-4-thiazolyl]ethenyl]benzylmethylacetat,
- α-(Methoxymethylen)-2-[(2-methyl-4-thiazolyl)methoxy]methylbenzylacetat.

**16.** Verfahren zur Herstellung der Verbindungen der Formel (I) nach Anspruch 1, **dadurch gekennzeichnet**, daß man eine Verbindung der Formel (II)

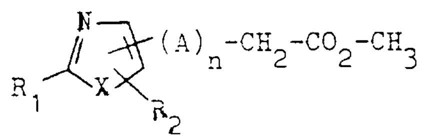

(II)

worin X, $R_1$, $R_2$, A und n wie vorstehend definiert sind, der Wirkung einer starken Base und eines Alkylformiats oder von N-Formylimidazol unterwirft, um eine Verbindung der Formel (III)

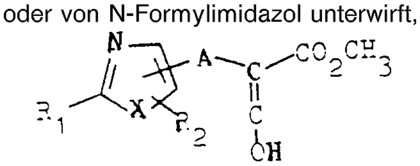

(III)

zu erhalten, die man der Wirkung eines Mittels mit der Fähigkeit, den $R_3$-Rest einzuführen, unterwirft, um die entsprechende Verbindung der Formel (I) zu erhalten, und daß man gegebenenfalls die verschiedenen Isomeren trennt.

**17.** Verfahren zur Herstellung der Verbindungen der Formel (I) nach Anspruch 1, worin der Rest A für eine Gruppierung ($A_1$) nach Anspruch 7 steht, **dadurch gekennzeichnet**, daß man eine Verbindung der Formel (IV)

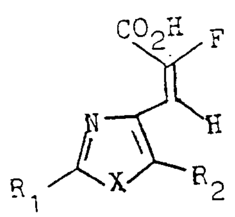

(IV)

worin X, $R_1$ und $R_2$ wie vorstehend definiert sind, oder ein funktionelles Derivat dieser Verbindung der Wirkung einer Verbindung der Formel (V)

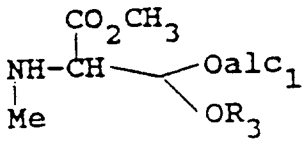

(V)

worin $R_3$ wie vorstehend definiert ist, und $alc_1$ für einen Alkylrest mit bis zu 8 Kohlenstoffatomen steht, unterwirft, um eine Verbindung der Formel (VI)

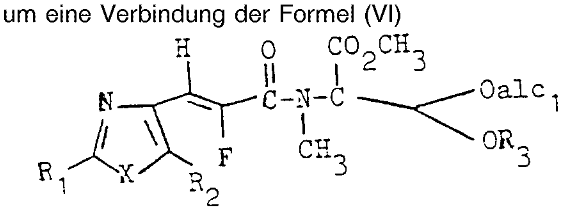

(VI)

zu erhalten, die man der Wirkung eines Mittels zur Abspaltung des Alkoholrestes $alc_1$-OH unterwirft, um die entsprechende Verbindung der Formel ($I_A$)

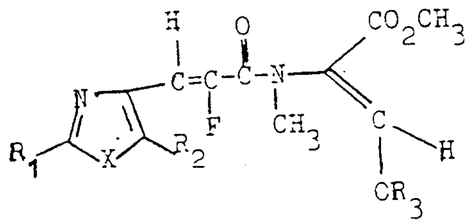

($I_A$)

zu erhalten.

**18.** Variante des Verfahrens nach Anspruch 16 zur Herstellung der Verbindungen der Formel (I), worin A für einen Rest

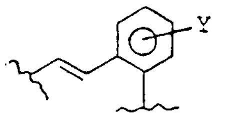

oder

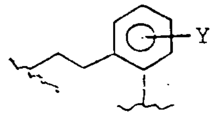

steht, **dadurch gekennzeichnet**, daß man nach der Wittig-Horner-Reaktion eine Verbindung der Formel (VII)

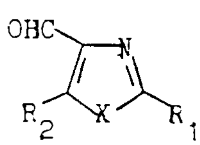

(VII)

worin X, $R_1$ und $R_2$ wie vorstehend definiert sind, der Wirkung einer Verbindung der Formel (VIII)

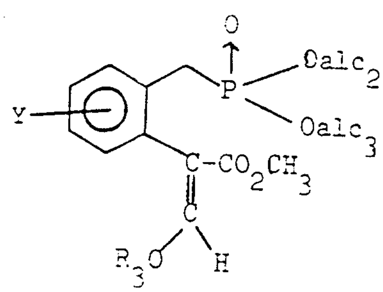

(VIII)

worin Y und $R_3$ wie vorstehend definiert sind, $alc_2$ und $alc_3$ gleich oder verschieden sind und für einen Alkylrest mit bis zu 8 Kohlenstoffatomen stehen, unterwirft, um eine entsprechende Verbindung der Formel ($I_B$)

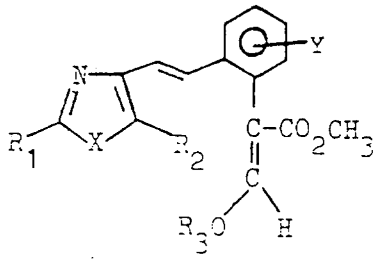

($I_B$)

zu erhalten, die man gegebenenfalls isoliert oder gegebenenfalls einer katalytischen Hydrierung unterwirft, um die Verbindungen der Formel ($I_C$)

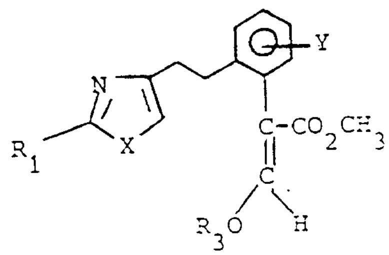

($I_C$)

zu erhalten.

**19.** Verbindungen der Formel (II) und (III) nach Anspruch 16, sowie Verbindungen (IV) und (VI) nach Anspruch 17.

**20.** Fungizide Präparate, umfassend als Wirkstoff mindestens eine Verbindung der Formel (I) nach einem der Ansprüche 1 bis 14.

**21.** Fungizide Präparate, umfassend als Wirkstoff mindestens eine Verbindung nach Anspruch 15.

**Patentansprüche für folgenden Vertragsstaat : ES**

**1.** Verfahren zur Herstellung von Verbindungen der allgemeinen Formel (I)

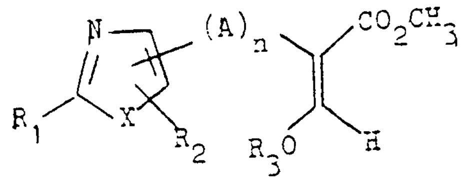

(I)

worin

- $R_1$ und $R_2$, die gleich oder verschieden sind, für ein Wasserstoffatom, ein Halogenatom, eine $CF_3$-Gruppe, einen Alkyl-, Alkenyl-, Alkinyl- oder Thioalkylrest mit bis zu 8 Kohlenstoffatomen, einen Alkoxy-, Thioalkoxy- oder $SO_2$-Alkylrest mit bis zu 8 Kohlenstoffatomen, einen Aryl-, Aryloxy- oder Thioarylrest mit bis zu 18 Kohlenstoffatomen, der gegebenenfalls substituiert ist, einen Heteroaryl- oder Heteroaryloxyrest, der gegebenenfalls substituiert ist, oder einen heterocyclischen, 5- oder 6-gliedrigen, gegebenenfalls substituierten Rest stehen,
- $R_3$ für einen Alkyl-, Alkenyl- oder Alkinylrest mit bis zu 8 Kohlenstoffatomen oder einen Arylrest mit bis zu 18 Kohlenstoffatomen oder einen Alkoxyalkoxyalkylrest mit bis zu 14 Kohlenstoffatomen steht,
- X für ein Sauerstoff- oder Schwefelatom oder einen $NR_4$-Rest steht, worin $R_4$ für ein Wasserstoffatom oder einen Alkyl-, Alkenyl- oder Alkinylrest, einen $SO_2$-Alkyl-, $SO_2$-Alkenyl- oder $SO_2$-

Alkinylrest mit bis zu 8 Kohlenstoffatomen oder einen Aryl- oder $SO_2$-Arylrest mit bis zu 18 Kohlenstoffatomen, der gegebenenfalls substituiert ist, steht,

- n den Wert 0 oder 1 hat,
- A für einen Rest ausgewählt aus der Gruppe der folgenden Reste

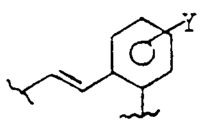 ; 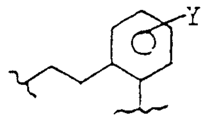 ; 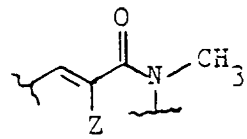 ; 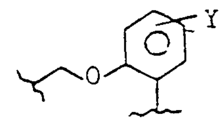

worin Y für ein Wasserstoffatom, ein Halogenatom, eine $CF_3$-Gruppe, einen Alkyl- oder Alkoxyrest mit bis zu 8 Kohlenstoffatomen steht, und Z für ein Wasserstoffatom oder ein Halogenatom steht, steht,

- die Geometrie der Doppelbindungen E oder Z oder ein Gemisch aus der Geometrie E und Z ist, **dadurch gekennzeichnet**, daß man eine Verbindung der Formel (II)

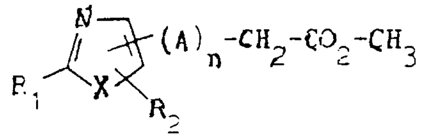

(II)

worin X, $R_1$, $R_2$, A und n wie vorstehend definiert sind, der Wirkung einer starken Base und eines Alkylformiats oder von N-Formylimidazol unterwirft, um eine Verbindung der Formel (III)

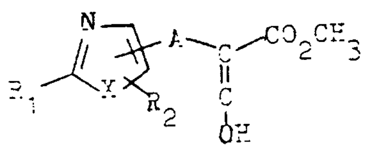

(III)

zu erhalten, die man der Wirkung eines Mittels mit der Fähigkeit, den $R_3$-Rest einzuführen, unterwirft, um die entsprechende Verbindung der Formel (I) zu erhalten, und daß man gegebenenfalls die verschiedenen Isomeren trennt.

**2.** Verfahren zur Herstellung der Verbindungen der Formel (I) nach Anspruch 1, **dadurch gekennzeichnet**, daß man

a) zur Herstellung von Verbindungen der Formel ($I_A$), entsprechend der Formel (I), worin A für eine Gruppierung $A_1$

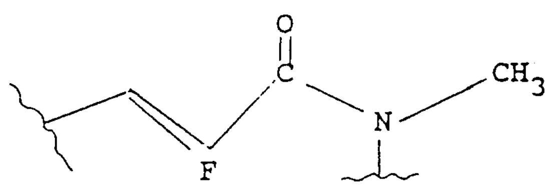

steht,
eine Verbindung der Formel (IV)

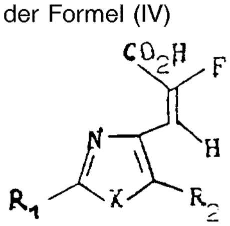

(IV)

worin X, $R_1$ und $R_2$ wie vorstehend definiert sind, oder ein funktionelles Derivat dieser Verbindung der Wirkung einer Verbindung der Formel (V)

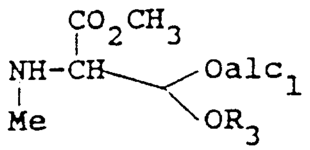

(V)

worin $R_3$ wie vorstehend definiert ist, und $alc_1$ für einen Alkylrest mit bis zu 8 Kohlenstoffatomen steht, unterwirft, um eine Verbindung der Formel (VI)

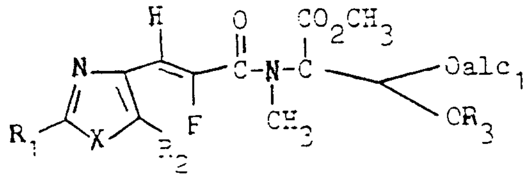

(VI)

zu erhalten, die man der Wirkung eines Mittels zur Abspaltung des Alkoholrestes $alc_1$-OH unterwirft, um die entsprechende Verbindung der Formel ($I_A$)

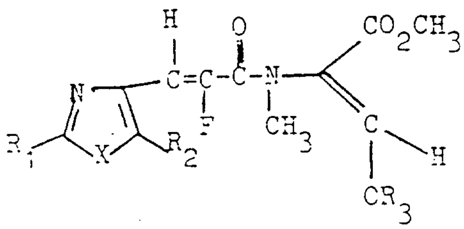

($I_A$)

zu erhalten, und daß man gegebenenfalls die Doppelbindungsisomeren trennt;

b) zur Herstellung von Verbindungen der Formel ($I_B$), entsprechend der Formel (I), worin A für eine Gruppierung

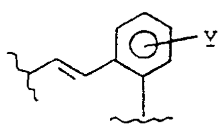

steht, oder der Formel ($I_C$), entsprechend der Formel (I), worin A für eine Gruppierung

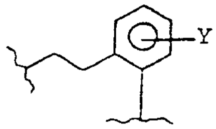

steht,

nach der Wittig-Horner-Reaktion eine Verbindung der Formel (VII)

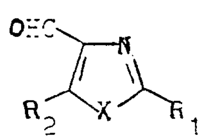

(VII)

worin X, $R_1$ und $R_2$ wie vorstehend definiert sind, der Wirkung einer Verbindung der Formel (VIII)

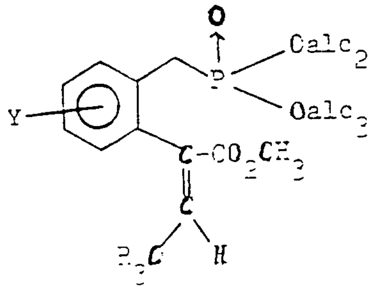

(VIII)

worin Y und $R_3$ wie vorstehend definiert sind, $alc_2$ und $alc_3$ gleich oder verschieden sind und für einen Alkylrest mit bis zu 8 Kohlenstoffatomen stehen, unterwirft, um die entsprechende Verbindung der Formel ($I_B$)

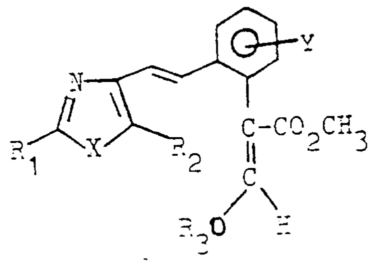

($I_B$)

zu erhalten, die man gegebenenfalls isoliert oder gegebenenfalls einer katalytischen Hydrierung unterwirft, um die Verbindungen der Formel ($I_C$)

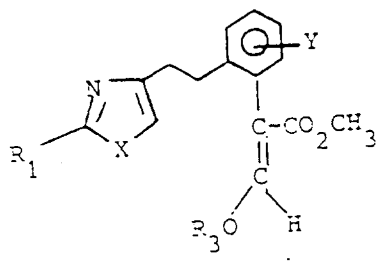

($I_C$)

zu erhalten, und daß man gegebenenfalls die Doppelbindungsisomeren trennt.

3. Verfahren nach Anspruch 2 zur Herstellung der Verbindungen der Formel ($I_A$) nach Anspruch 2, **dadurch gekennzeichnet**, daß man eine Verbindung der Formel (IV) nach Anspruch 2 oder ein funktionelles Derivat dieser Verbindung der Wirkung einer Verbindung der Formel (V) nach Anspruch 2 unterwirft, um die Verbindung der Formel (VI) zu erhalten, die man der Wirkung eines Mittels zur Abspaltung des Alkohols $alc_1$-OH unterwirft, um die entsprechende Verbindung der Formel ($I_A$) zu erhalten, und daß man gegebenenfalls die Doppelbindungsisomeren trennt.

4. Verfahren nach Anspruch 2 zur Herstellung der Verbindungen der Formel ($I_B$) oder ($I_C$) nach Anspruch 2, **dadurch gekennzeichnet**, daß man nach der Wittig-Horner-Reaktion eine Verbindung der Formel (VII) nach Anspruch 2 der Wirkung einer Verbindung der Formel (VIII) nach Anspruch 2 unterwirft, um die Verbindung der Formel ($I_B$) zu erhalten, die man gegebenenfalls isoliert, und daß man gegebenenfalls die Doppelbindungsisomeren trennt, oder sie gegebenenfalls einer katalytischen Hydrierung unterwirft, um die Verbindung der Formel ($I_C$) zu erhalten, und gegebenenfalls die Doppelbindungsisomeren trennt.

5. Verfahren nach Anspruch 1 oder 2, **dadurch gekennzeichnet**, daß man als Ausgangsverbindung eine Verbindung der Formel (II), (IV) oder (VII) verwendet, worin X für ein Schwefelatom steht.

6. Verfahren nach Anspruch 2 oder 4, **dadurch gekennzeichnet**, daß man als Ausgangsverbindung eine Verbindung der Formel (VIII) verwendet, worin Y für ein Wasserstoffatom steht.

**7.** Verfahren nach Anspruch 1, **dadurch gekennzeichnet**, daß man als Ausgangsverbindung eine Verbindung der Formel (II) verwendet, worin A für einen Rest

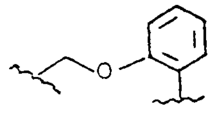

steht.

**8.** Verfahren nach Anspruch 1, **dadurch gekennzeichnet**, daß man als Ausgangsverbindung eine Verbindung der Formel (II) verwendet, worin A für einen Rest

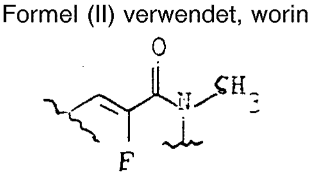

$(A_1)$

oder

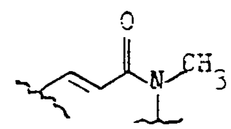

$(A_2)$

steht.

**9.** Verfahren nach Anspruch 1, **dadurch gekennzeichnet**, daß man als Ausgangsverbindung eine Verbindung der Formel (II) verwendet, worin n den Wert 0 besitzt.

**10.** Verfahren nach einem der Ansprüche 1 bis 9, **dadurch gekennzeichnet**, daß man als Ausgangsverbindung eine Verbindung der Formel (II), (IV) oder (VII) verwendet, worin $R_1$ für ein Wasserstoffatom, ein Chloratom, einen Alkyl- oder Alkenylrest mit bis zu 5 Kohlenstoffatomen, einen Alkoxyrest mit bis zu 5 Kohlenstoffatomen oder einen Rest

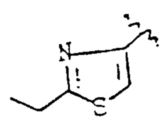

steht.

**11.** Verfahren nach einem der Ansprüche 1 bis 9, **dadurch gekennzeichnet**, daß man als Ausgangsverbindung eine Verbindung der Formel (II), (IV) oder (VII) verwendet, worin $R_2$ für ein Wasserstoffatom steht.

**12.** Verfahren nach einem der Ansprüche 1 bis 9, **dadurch gekennzeichnet**, daß man als Ausgangsverbindung eine Verbindung der Formel (II), (IV) oder (VII) verwendet, worin $R_1$ und/oder $R_2$ gegebenenfalls substituierte Arylreste sind.

**13.** Verfahren nach einem der Ansprüche 1 bis 9, **dadurch gekennzeichnet**, daß man als Ausgangsverbindung eine Verbindung der Formel (II), (IV) oder (VII) verwendet, worin $R_1$ eine $SCH_3$-Gruppe ist, und $R_2$ eine $CH_3$-Gruppe ist.

**14.** Verfahren nach Anspruch 12 oder 13, **dadurch gekennzeichnet**, daß man als Ausgangsverbindung eine Verbindung der Formel (II) verwendet, bei der der Alkoxyacrylatrest in Position 5 steht.

**15.** Verfahren nach Anspruch 1 oder 2, **dadurch gekennzeichnet**, daß man eine der nachstehenden Verbindungen herstellt:

- (E,Z)-3-Methoxy-[2-methyl-[1-oxo-3-(4-thiazolyl)-2-propenyl]amino]-2-methylpropenoat,
- 2-(2-Methylthio-4-methyl-5-thiazolyl)-3-methoxy-2-methylpropenoat,
- 2-[N-[3-[2'-ethyl-(2,4'-bisthiazol)-4-yl]-1-oxo-2-(E)-propenyl]methylamino]-3-(Z)-methoxymethylpropenoat,

- 2-[N-[3-[2'-ethyl-(2,4'-bisthiazol)-4-yl]-1-oxo-2-(Z)-fluorpropenyl]methylamino]-3-methoxy-(Z)-methylpropenoat,
- (Z,Z)-2-[(2-Fluor-1-oxo-3-(4-thiazolyl)-2-propenyl)-N-methylamino]-3-methoxy-2-methylpropenoat,
- (E,E)-α-(Methoxymethylen)-2-[2-(4-thiazolyl)ethenyl]benzylmethylacetat,
- 2-(E)-[[[2'-ethyl-(2,4'-bisthiazol)-4-yl]ethenyl]-2-phenyl]-3-methoxy-2-(E)-methylpropenoat,
- (E,Z)-3-Methoxy-2-[methyl-[1-oxo-3-[2-(trifluormethyl)-4-thiazolyl]-2-propenyl]amino]-2-methylpropenoat,
- α-(Methoxymethylen)-2-[(2-phenyl-4-thiazolyl)methoxy]benzylmethylacetat,
- (E)-2-[[2'-ethyl-(2,4'-bisthiazol)-4-yl]methoxy]-α-(methoxymethylen)benzylmethylacetat,
- α-(E)-(Methoxymethylen)-2-(E)-[[2-(2-pentyl)-4-thiazolyl]ethenyl]benzylmethylacetat,
- (E)-α-(Methoxymethylen)-2-[(4-thiazolyl-2-methylethyl]methoxy]benzylmethylacetat,
- E,E-α-(Methoxymethylen)-2-[2-(2-methylethyl-4-thiazolyl)ethenyl]benzylmethylacetat,
- E,Z-α-(Methoxymethylen)-2-[2-(2-methylethyl-4-thiazolyl)ethenyl]benzylmethylacetat,
- α-(Z)-(Methoxymethylen)-2-(E)-[[2-(2-pentyl)-4-thiazolyl]ethenyl]benzylmethylacetat,
- α-(Methoxymethylen)-2-[(2-methyl-4-thiazolyl)methoxy]methylbenzylacetat.

**16.** Verfahren zur Herstellung von fungiziden Präparaten, **dadurch gekennzeichnet**, daß man als Wirkstoff mindestens eine Verbindung der Formel (I) nach einem der Ansprüche 1 bis 14 formuliert.

**17.** Verfahren zur Herstellung von fungiziden Präparaten, **dadurch gekennzeichnet**, daß man als Wirkstoff mindestens eine Verbindung der Formel (I) nach Anspruch 15 formuliert.